# EUROPEAN PATENT SPECIFICATION

(11) **EP 3 106 522 B1**
(45) Date of publication and mention of the grant of the patent: **22.09.2021**
(21) Application number: 15746275.5
(22) Date of filing: 09.02.2015
(51) Int. Cl.: C12P 7/22, C12N 9/02, C12P 7/24, C12N 1/20, C12N 15/09

(54) **METHOD FOR PRODUCING PHENYL PROPANE-BASED COMPOUNDS USING ENZYMES**
VERFAHREN ZUR HERSTELLUNG VON VERBINDUNGEN AUF PHENYLPROPANBASIS UNTER VERWENDUNG VON ENZYMEN
PROCÉDÉ DE PRODUCTION D'UN COMPOSÉ DE TYPE PHÉNYLE PROPANE METTANT EN OEUVRE DES ENZYMES

(30) Priority: 10.02.2014 JP 2014023843
(43) Date of publication of application: 21.12.2016
(73) Proprietor: Japan Agency for Marine-Earth Science and Technology, Yokosuka-shi, Kanagawa 237-0061 (JP)
(72) Inventor: OHTA, Yukari, Yokosuka-shi Kanagawa 237-0061 (JP); HATADA, Yuji, Yokosuka-shi Kanagawa 237-0061 (JP); HASEGAWA, Ryoichi, Hyogo 6660035 (JP); NISHI, Shinro, Yokosuka-shi Kanagawa 237-0061 (JP)
(74) Representative: Schulz Junghans Patentanwälte PartGmbB
(86) International application number: PCT/JP2015/053551
(87) International publication number: WO 2015/119280

(56) References cited:
- EP-A1- 2 218 773
- JP-A- 2002 034 557
- JP-A- 2006 230 255
- JP-A- 2012 130 326
- US-A1- 2012 196 334
- GALL, D.L. ET AL.: 'Stereochemical Features of Glutathione-dependent Enzymes in the Sphingobium sp. Strain SYK-6 beta-Aryl Etherase Pathway' THE JOURNAL OF BIOLOGICAL CHEMISTRY vol. 289, no. 12, pages 8656 - 8667, XP055218446
- AKIHITO OCHIAI: 'Sphingomonas wa Yutosei' JOURNAL OF THE SOCIETY FOR BIOSCIENCE AND BIOENGINEERING, JAPAN vol. 89, no. 10, October 2011, page 614, XP008182268
- TAKAO KISHIMOTO: 'Synthesis of Lignin Model Compounds and Their Application to Wood Research' JOURNAL OF WOOD SCIENCE vol. 55, no. 4, 2009, pages 187 - 197, XP055218459
- MASAI, E. ET AL.: 'Complete Genome Sequence of Sphingobium sp. Strain SYK-6, a Degrader of Lignin-Derived Biaryls and Monoaryls' JOURNAL OF BACTERIOLOGY vol. 194, no. 2, January 2012, pages 534 - 535, XP055218448
- YUKARI OTA ET AL.: 'Shinkai Chinboku kara Tanri shita Bacteria no Bunruigakuteki Ichi to Hokozoku Monomer Taisha' DAI 57 KAI PROCEEDINGS OF THE LIGNIN SYMPOSIUM 01 October 2012, page 128, XP008182505
- GALL, D.L. ET AL.: 'A Group of Sequence-Related Sphingomonad Enzymes Catalyzes Cleavage of beta- Aryl Ether Linkages in Lignin beta-Guaiacyl and beta- Syringyl Ether Dimers' ENVIRON. SCI. TECHNOL., 2014.09 vol. 48, pages 12454 - 12463, XP055218461
- KAGAMI IIDA ET AL.: 'Shinkai Chinboku kara Tanri shita Novosphingobium-zoku Saikin no 2 Ryotai Lignin Model Kagobutsu beta-0-4 Ketsugo Kangenteki Kairetsu Kosogun no Kaiseki' DAI 64 KAI ABSTRACTS OF THE ANNUAL MEETING OF THE JAPAN WOOD RESEARCH SOCIETY 03 March 2014, XP008182386
- KANAKO KUROSAWA ET AL.: 'Shinkai Yurai Novosphingobium-zoku Saikin o Mochiita Biomass kara no Hokozoku Monomer Seisan' DAI 64 KAI ABSTRACTS OF THE ANNUAL MEETING OF THE JAPAN WOOD RESEARCH SOCIETY 03 March 2014, XP008182384
- YUKARI OTA ET AL.: 'Shinkai Chinboku kara Tanri shita Biseibutsu ga Seisan suru Lignin Yurai Hokozoku Monomer Genryo to shite no Potential' DAI 64 KAI ABSTRACTS OF THE ANNUAL MEETING OF THE JAPAN WOOD RESEARCH SOCIETY 03 March 2014, XP008182385
- OHTA, Y. ET AL.: 'Draft Genome Sequence of Novosphingobium sp. Strain MBES04, Isolated from Sunken Wood from Suruga Bay, Japan' GENOME ANNOUNCEMENTS vol. 3, no. ISSUE, 15 January 2015, pages E01373 - 14, XP055218472
- AN: "short-chain dehydrogenase/reductase SDR [Novosphingobium aromaticivorans DSM 12444]", NCBI,, 23 July 2008 (2008-07-23), XP008156902,
- DATABASE UniProt [Online] 27 July 2011 (2011-07-27), "SubName: Full=Short-chain dehydrogenase/reductase SDR {ECO:0000313|EMBL:CCA92080.1};", retrieved from EBI accession no. UNIPROT:F6IKX8 Database accession no. F6IKX8

## Description

### TECHNICAL FIELD

This invention relates to a method for specifically producing a compound having a phenyl propane structure from natural biomass containing lignins and lignin-related substances by using enzymes and also to enzymes to be used for the method.

### BACKGROUND ART

Lignins are amorphous polymeric substances existing in plants as ingredients of vascular bundle cell walls. Lignins are formed as a result of complex condensation reactions of phenyl propane-based constituent units and show a remarkable chemical structural characteristic of containing methoxy groups. Lignins take a role of causing lignified plant cells to mutually agglutinate, thereby strengthening plant tissues. Lignins are contained by 18 to 36% in woods and by 15 to 25% in herbaceous plants. Thus, various attempts have been and being made to degrade lignins and obtain useful compounds therefrom for the purpose of effectively exploiting wood resources.

Meanwhile, known phenyl propane-based compounds include coumaric acid, cinnamic acid, caffeic acid (3,4-dihydorxy-cinnamic acid), eugenol, anethol, coniferyl alcohol, sinapyl alcohol and ferulic acid. In industrial fields, phenyl propane-based compounds are useful because such compounds can be used for medicines, functional foods and synthetic intermediates of various chemical products such as perfumes, spices, essential oils, fungicides, anesthetics and antioxidant agents.

For example, methods for non-specifically degrading lignins contained in lignocellulose substances of woods, rice straws and so on down to low molecules by using physiochemical techniques such as gasifying techniques involving high-temperature high-pressure processing (see Patent Documents 1 and 2) and pressurized hot water treatment techniques (see Patent Document 3)are known. However, with any of the above listed techniques, it is very difficult to produce a specific compound from lignins. In other words, when any of the above listed techniques is employed, phenyl propane-based compounds having three carbon atoms in each carbon side chain that is directly bonded to a benzene ring skeleton, which compounds operate as unit structures of lignins, are further degraded to lower molecules. To be more accurate, phenyl propane-based compounds are non-specifically transformed into phenols such as guaiacols, sylingols and so on where one or more than one carbon side chains having no carbon atoms are directly bonded to a benzene ring skeleton and also into phenyl methane compounds such as vanillin, syringaldehyde and so on where one or more than one carbon side chains having one carbon atoms are directly bonded to a benzene ring skeleton. Additionally, degradation products of lignins are mixtures of a variety of components and hence it is highly difficult to obtain only phenyl propane-based compounds therefrom. In other words, it is not possible to specifically produce phenyl propane-based compounds with any of the above identified techniques. Furthermore, if any of the above listed physiochemical methods is adopted and put to practical use, it requires a vast amount of energy and special apparatus.

In view of the above-identified problems, attempts are being made to obtain degradation products of lignins by biologically processing lignins. For example, a method of degrading lignins by inoculating white-rot fungus into lignocellulose substances and incubating them there is known (see Patent Document 4).

Meanwhile, since it is known that β-aryl ether type bonds account for about 50% of the chemical bonds existing in natural lignins, if β-aryl ether bonds can be cleaved or not is highly significant from the viewpoint of degrading natural lignins. Known microorganisms that produce enzymes capable of specifically cleaving β-aryl ether type bonds include microorganisms of the genus Sphingobium (see Patent Document 5 and Non-Patent Document 1; note, however, microorganisms of that genus are described as those the genus Pseudomonas in Non-Patent Document 1), microorganisms of the genus Brevundimonas (see Patent Document 6) and microorganisms of the genus Pseudomonas (see Non-Patent Document 2). US2012196334 discloses certain lignine degrading enzyme activities of Novosphingobium aromaticivorans and Novosphingobium sp. PPY1 identified by Blast search.

Patent Documents 4 through 6 and Non-Patent Documents 1 and 2 describe microorganisms of the genus Sphingobium, microorganisms of the genus Brevundimonas and microorganisms of the genus Pseudomonas as well as methods of producing phenyl propane-based compounds by cleaving β-aryl ether type bonds of guaiacylglycerol-β-guaiacyl ether or 3-hydroxy-2-(2-methoxyphenoxy)-1-(3-methoxy-4-hydroxyphenyl)-1-propanone, which are model compounds of lignins, by means of enzymes capable of cleaving β-aryl ether type bonds that are produced by microorganisms of any of the above listed genera.

Additionally, Non-Patent Document 3 describes that, when producing phenyl propane-based compounds from guaiacylglycerol-β-guaiacyl ether by means of enzymes capable of cleaving β-aryl ether type bonds that are derived from microorganisms of the genus Sphingobium, the intended production of phenyl propane-based compound can be realized by means of 3-stage enzyme-involving sequential reactions including the first reaction of forming carbonyl groups by causing such enzymes to act on the Cα carbon of guaiacylglycerol-β-guaiacyl ether and the alcoholic hydroxyl group bonded to the carbon atom, the second reaction of cleaving the β-O-4 ether bond existing in the produced carbonyl compound and the third reaction of removing the glutathione from the gluthathione-containing intermediate produced as a result of the second reaction.

### PRIOR ART DOCUMENTS

### PATENT DOCUMENTS

Patent Document 1: JP 2012-140346 A
Patent Document 2: JP 2012-50924 A
Patent Document 3: JP 2010-239913 A
Patent Document 4: JP 1975-46903 A
Patent Document 5: JP 1993-336976 A
Patent Document 6: JP 2002-34557 A

### NON-PATENT DOCUMENTS

Non-Patent Document 1: FEBS Lett. 249 (2), 1989, pp. 348-352
Non-Patent Document 2: Mokuzai Gakkaishi, Vol. 31 (11), 1985, pp. 956-958
Non-Patent Document 3: Biosci Biotechnol Biochem. 2011; 75 (12):2404-7

### SUMMARY OF THE INVENTION

### PROBLEMS TO BE SOLVED BY THE INVENTION

According to the description of Patent Document 4, the method described in the document can provide possibility of degrading lignins by causing microorganisms to act on the lignins to be degraded. However, when white-rot fungi and ligninase that white-rot fungi produce are caused to act on lignocellulose substances, not only the products lack structural uniformity but also polymerization reactions mainly of the products progress because the specificity of the lignin degrading reaction caused by the microorganisms is very low. Therefore, the method described in Patent Document 4 has a problem that the reaction products lack usefulness as industrial raw materials.

The methods described in Patent Documents 5 and 6 and Non-Patent Documents 1 through 3 utilize microorganisms and enzymes produced by microorganisms of the type under consideration to produce phenyl propane-based compounds from model compounds of lignin. However, these documents do not describe any fact that phenyl propane-based compounds are obtained from natural biomass by causing microorganisms and enzymes to act on model compounds of lignin. In particular, it is not possible to produce phenyl propane-based compounds only by using the enzymes described in Patent Document 5. It is not possible either to produce phenyl propane-based compounds by using the microorganisms described in Non-Patent Document 1 because those microorganisms completely break down guaiacylglycerol-β-guaiacyl ether down to CO₂ for anabolism.

Since chemical structures like the structure of guaiacylglycerol-β-guaiacyl ether, where an OH group is bonded to the α carbon of a β-aryl ether type bond, are frequently observable in natural biomass, it is very difficult to degrade natural biomass by using microorganisms and enzymes produced by microorganisms of the type under consideration as described in Patent Document 6. In other words, it is not possible to produce phenyl propane-based compounds from guaiacylglycerol-β-guaiacyl ether by using enzymes that are derived from microorganisms of the genus Brevundimonas and capable of cleaving β-aryl ether type bonds. Therefore, enzymes as described in Patent Document 6 are accompanied by a problem that such enzymes can hardly act on natural biomass where Cα carbons of β-aryl ether type bonds often do not participate in forming carbonyl groups.

Furthermore, Patent Document 6 describes that enzymes that are derived from microorganisms of the genus Brevundimonas and capable of cleaving β-aryl ether type bonds specifically and reductively cleave the arylglycerol-β-aryl ether type bond of 1-(4-benzyloxy-3-methoxyphenyl)-3-hydroxy-2-(2-methoxyphenoxy)propane-1-on (Compound IX described in Patent Document 6) and also cleave β-aryl ether type bonds regardless of existence or non-existence of one or more than one free phenolic hydroxyl groups at the aryl glycerol part of the β-aryl ether type bond. However, the level of activity of such enzymes relative to the above identified compound is of the order of one-hundredth of the level of activity thereof relative to 1-(4-hydroxy-3-methoxyphenyl)-3-hydroxy-2-(2-methoxyphenoxy)propane-1-on and hence far from a satisfactory level.

Production of enzymes as described in Patent Document 6 by cultivation requires setting cumbersome cultivation conditions such as addition of one or more than one derivative substances. Enzyme gene information for mass production of enzymes by using popular host genes for genetic recombination has not been obtained. Each of 1-(4-hydroxy-3-methoxyphenyl)-3-hydroxy-2-(2-methoxyphenoxy)propane-1-on and its derivative 1-(4-benzyloxy-3-methoxyphenyl)-3-hydroxy-2-(2-methoxyphenoxy)propane-1-on has two optical isomers on which enzymes that are derived from microorganisms of the genus Brevundimonas and capable of cleaving β-aryl ether type bonds act but Patent Document 6 does not describe at all about the activity of such enzymes relative to each of the optical isomers.

While microorganisms described in Non-Patent Document 2 are capable of metabolizing guaiacylglycerol-β-guaiacyl ether, the yield of the obtained product is very low because the produced phenyl propane-based compounds are further metabolized and transformed into different compounds.

Enzymes described in Non-Patent Document 3 can be obtained by using only αR, βS optical isomer and αS, βS optical isomer of guaiacylglycerol-β-guaiacyl ether, which has four optical isomers (αS, βR; αR, β5; αR, βR; αS, βS), as starting material but αS, βR and αR, βR optical isomers of guaiacylglycerol-β-guaiacyl ether cannot be used as starting material. Then, as a result, there arises a problem that the yield of obtained phenyl propane-based compounds is low. Additionally, with regard to substrates where enzymes as described in Non-Patent Document 3 act, only degradation of compounds having free phenolic hydroxyl groups is known and Non-Patent Document 3 does not describe at all about compounds having free phenolic hydroxyl groups.

Thus, the problem to be solved by the present invention is to provide a method which, if compared with the methods described in Patent Documents 5 and 6 and Non-Patent Documents 1 through 3, more specifically and efficiently produces a compound having a phenol propane structure from natural biomass containing lignins by causing enzymes to act on the biomass.

### MEANS FOR SOLVING THE PROBLEMS

The inventors of the present invention have paid intensive research efforts to solve the above identified problem and succeeded in deciphering genome sequence information on Novosphingobium sp. MBES04 strain, which is a microorganism obtained by degrading lignins isolated from woods sunken in deep seas, and acquiring a group of genes of a series of enzymes that participate in specifically producing compounds having a phenyl propane structure from natural biomass containing lignins on the basis of the obtained information. Furthermore, as a result of preparing a recombinant enzyme group from the acquired group of genes and analyzing the degradation style of the group of genes, using lignings-containing biomass as substrate, the inventors of the present invention found six strains of enzyme that can specifically produce phenyl propane-based compounds at a high yield. The present invention was achieved on the basis of these successful researches and findings.

Thus, the present invention provides a method for producing a phenyl propane-based compound comprising a step of producing a phenyl propane-based compound by causing enzymes derived from microorganisms of the genus Novosphingobium to act on biomass containing lignins and/or lignin-related substances in the presence of NAD and reduction type glutathione. In the production method of the present invention, the microorganisms of the genus Novosphingobium are Novosphingobium species MBES04 (deposition number: NITE AP-01797). In the production method of the present invention, the enzymes are a combination of enzymes described in (1) shown below and enzymes described in (2) shown below as well as enzymes described in (3) shown below and/or enzymes described in (4) shown below.
(1) Short-chain dehydrogenase/reductase that belongs to the Rossmann-fold NAD(P)(+) binding protein super family, has Multi-domain of PRK06194 and oxidizes the Cα carbon of guaiacylglycerol-β-guaiacyl ether
(2) glutathione S-transferase that has Multi-domain of PRK 15113 and maiA at the side of Gst and the N-end
(3) glutathione S-transferase that has Multi-domain of PRK 10387 and maiA at the side of Gst and the N-end
(4) glutathione S-transferase that has Multi-domain of Gst, PRK11752, PRK15113 and maiA. In the production method of the present invention, the enzyme of (1) is either C10G0069 having an amino acid sequence as defined in the sequence listing with sequence number 1 or C10G0093 having an amino acid sequence as defined in the sequence listing with sequence number 2; the enzyme of (2) is either C10G0076 having an amino acid sequence as defined in the sequence listing with sequence number 3 or C10G0077 having an amino acid sequence as defined in the sequence listing with sequence number 4; the enzyme of (3) is C10G0078 having an amino acid sequence as defined in the sequence listing with sequence number 5; and the enzyme of (4) is C10G0075 having an amino acid sequence as defined in the sequence listing with sequence number 6. The phenyl propane-based compound is expressed by general formula (A) 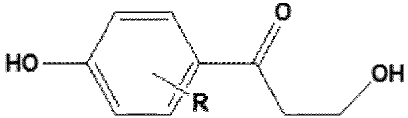 where R represents 1, 2 or more alkyl groups or alkoxy groups having 1 to 5 carbon atoms or hydrogen atoms.

Preferably, in the production method of the present invention, the phenyl propane-based compound is one selected from a group of 3-hydroxy-1-(4-hydroxy-3-methoxyphenyl)-1-propanone, 3-hydroxy-1-(4-hydroxy-3,5-dimethoxyphenyl)-1-propanone and 3-hydroxy-1-(4-hydroxyphenyl)-1-propanone.

Preferably, in the production method of the present invention, the phenyl propane-based compound is 3-hydroxy-1-(4-hydroxy-3-methoxyphenyl)-1-propanone.

Also disclosed is a method of producing a carbonyl phenyl-based compound comprising a step of obtaining a carbonyl phenyl-based compound by causing enzymes derived from microorganisms of the genus Novosphingobium as described in (1) below to act on biomass containing lignins and/or lignin-related substances in the presence of NAD.
(1) Short-chain dehydrogenase/reductase that belongs to the Rossmann-fold NAD(P)(+) binding protein super family, has Multi-domain of PRK06194 and oxidizes the Cα carbon of guaiacylglycerol-β-guaiacyl ether

Preferably, in the production method of the present invention as defined above, the enzyme of (1) is either C10G0069 having an amino acid sequence as defined in the sequence listing with sequence number 1 or C10G0093 having an amino acid sequence as defined in the sequence listing with sequence number 2.

In still another aspect of the present invention, there is provided short-chain dehydrogenase/reductase that is derived from microorganisms of the genus Novosphingobium and belongs to the Rossmann-fold NAD(P)(+) binding protein super family, while it has Multi-domain of PRK06194 and oxidizes the Cα carbon of guaiacylglycerol-β-guaiacyl ether. The microorganisms of the genus Novosphingobium are Novosphingobium species MBES04 (deposition number: NITE AP-01797). The short-chain dehydrogenase/reductase is either C10G0069 having an amino acid sequence as defined in the sequence listing with sequence number 1 or C10G0093 having an amino acid sequence as defined in the sequence listing with sequence number 2.

Preferably, in the enzyme of the present invention as defined above, the short-chain dehydrogenase/reductase has a molecular weight of 34kDa, which is confirmable by means of SDS-PAGE, and its optimum pH and optimum temperature are respectively between 8.5 and 9.5 and between 10 and 15°C.

Preferably, in the enzyme of the present invention as defined above, the short-chain dehydrogenase/reductase has a molecular weight of 34kDa, which is confirmable by means of SDS-PAGE, and its optimum pH and optimum temperature are respectively between 8.5 and 10.5 and between 25 and 30°C.

### ADVANTAGES OF THE INVENTION

Thus, the production method and the enzyme according to the present invention can specifically and efficiently produce a useful compound having a phenol propane structure from natural biomass containing lignins and lignin-related substances that are originating from natural substances such as agricultural wastes and woods.

### BREIF DESCRIPTION OF THE DRAWINGS

FIG. 1 is a schematic illustration of the relationship between a mode of existence of an enzyme group that can be used for the production method of the present invention and optical isomers of the substrate where such enzymes act.
FIG. 2 is a schematic illustration of some of the results obtained by a DELTA-BLAST search, using an amino acid sequence that gene c10g0069 encodes as query for the search.
FIG. 3 is a schematic illustration of some of the results obtained by a DELTA-BLAST search, using an amino acid sequence that gene c10g0093 encodes as query for the search.
FIG. 4 is a schematic illustration of some of the results obtained by a DELTA-BLAST search, using an amino acid sequence that gene c10g0076 encodes as query for the search.
FIG. 5 is a schematic illustration of some of the results obtained by a DELTA-BLAST search, using an amino acid sequence that gene c10g0077 encodes as query for the search.
FIG. 6 is a schematic illustration of some of the results obtained by a DELTA-BLAST search, using an amino acid sequence that gene c10g0078 encodes as query for the search.

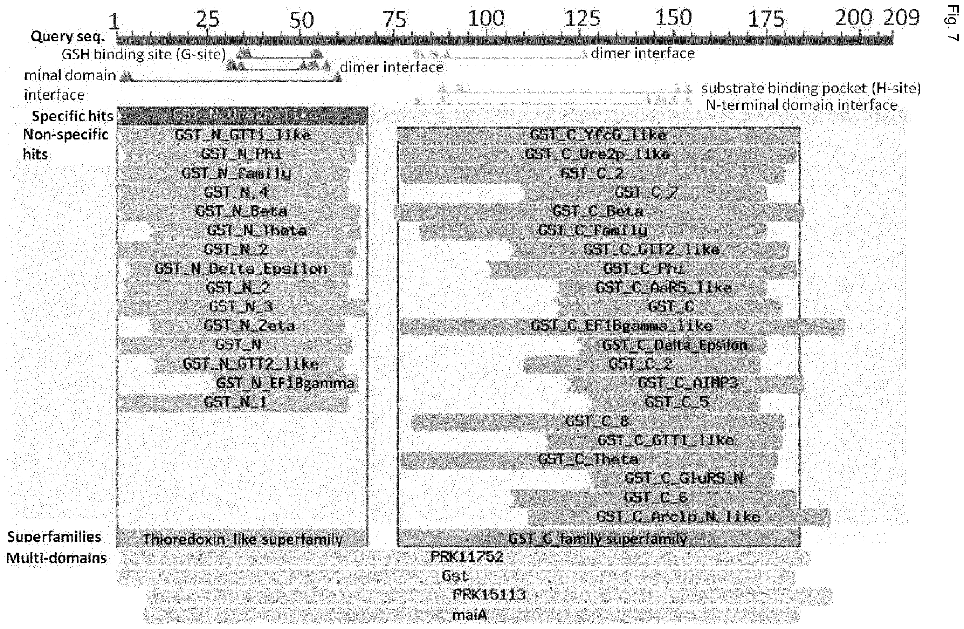

FIG. 7 is a schematic illustration of some of the results obtained by a DELTA-BLAST search, using an amino acid sequence that gene c10g0075 encodes as query for the search.
FIG. 8 is a graph showing some of the results obtained by evaluating the optimum temperature of C10G0069.
FIG. 9 is a graph showing some of the results obtained by evaluating the optimum pH of C10G0069.
FIG. 10 is a graph showing some of the results obtained by evaluating the optimum temperature of C10G0093.
FIG. 11 is a graph showing some of the results obtained by evaluating the optimum pH of C10G0093.
FIG. 12 is a graph showing some of the results obtained by evaluating the optimum temperature of C10G0076.
FIG. 13 is a graph showing some of the results obtained by evaluating the optimum pH of C10G0076.
FIG. 14 is a graph showing some of the results obtained by evaluating the optimum temperature of C10G0077.
FIG. 15 is a graph showing some of the results obtained by evaluating the optimum pH of C10G0077.

### MODES FOR CARRYING OUT THE INVENTION

Now, the present invention will be described in greater detail below.

The production method of the present invention is a method for producing a phenyl propane-based compound comprising a step of producing a phenyl propane-based compound by causing enzymes derived from microorganisms of the genus Novosphingobium to act on biomass containing lignins and/or lignin-related substances in the presence of NAD and reduction type glutathione.

With the above defined production method of the present invention, as lignins and lignin-related substances contained in biomass are subjected to the action of enzymes derived from microorganisms of the genus Novosphingobium, a phenyl propane-based compound such as 3-hydroxy-1-(4-hydroxy-3-methoxyphenyl)-1-propanone, 3-hydroxy-1-(4-hydroxy-3,5-dimethoxyphenyl)-1-propanone, 3-hydroxy-1-(4-hydroxyphenyl)-1-propanone or the like is produced.

A phenyl propane-based compound that can be obtained by the above defined production method of the present invention may be a compound expressed by general formula (A) shown below: 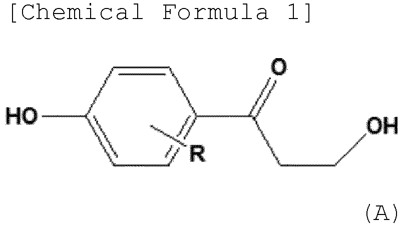 (where R represents 1 or more than 2 alkyl groups or alkoxy groups having 1 to 5 carbon atoms or hydrogen atoms) that has a carbonyl group at 1-position and hydroxyl groups respectively at 3-position and at 4-position of a phenyl group. More specifically, the compound is 3-hydroxy-1-(4-hydroxy-3-methoxyphenyl)-1-propanone, 3-hydroxy-1-(4-hydroxy-3,5-dimethoxyphenyl)-1-propanone or 3-hydroxy-1-(4-hydroxyphenyl)-1-propanone and, more preferably, it is 3-hydroxy-1-(4-hydroxy-3-methoxyphenyl)-1-propanone.

Of the above listed compounds, for example, 3-hydroxy-1-(4-hydroxy-3-methoxyphenyl)-1-propanone has a structure expressed by formula (I) shown below.

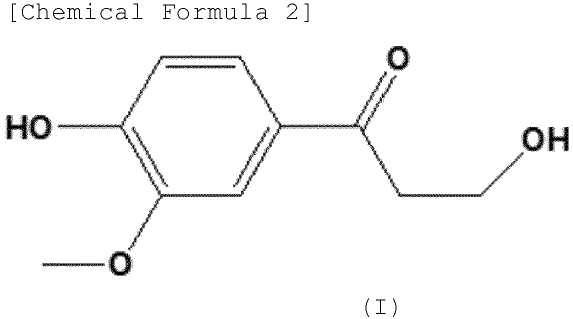

3-hydroxy-1-(4-hydroxy-3-methoxyphenyl)-1-propanone can qualitatively and quantitatively be analyzed, for example, by means of reverse phase HPLC. The conditions to be met for reverse phase HPLC include that an Octa Decyl Silyl group-modified silica gel column (ODS column) is to be employed along with eluent A (2mM ammonium acetate, 0.05%V/V formic acid) and eluent B (100%V/V methanol), that a column temperature of 40°C and a flow rate of 1.2 ml/min are to be set and that a mixture solution of eluent A 90%V/V and eluent B 10%V/V is to be fed for a minute and subsequently eluent B is to be fed at a gradient of 10%V/V to 95%V/V for seven minutes. As the above conditions are satisfied, 3-hydroxy-1-(4-hydroxy-3-methoxyphenyl)-1-propanone can be detected with a peak of retention time of around 4. 5 minutes by using a UV detector (270nm). It can be quantified by means of a calibration curve method, an internal standard method or the like provided that standard 3-hydroxy-1-(4-hydroxy-3-methoxyphenyl)-1-propanone is employed.

The above defined production method of the present invention employs biomass containing lignins and/or lignin-related substances. While lignins to be used as starting material are not subjected to any particular limitations so long as they are known to those who are skilled in the art, they are typically found in vascular bundles of plants and known to have a complicatedly polymerized dendritic structure where 3 types of phenylpropanoid that are expressed respectively by chemical formulas (B) through (D) shown below operate as unit constituents.

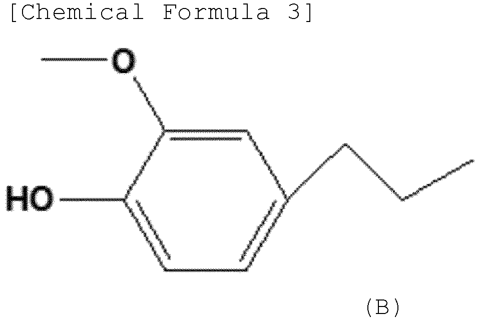

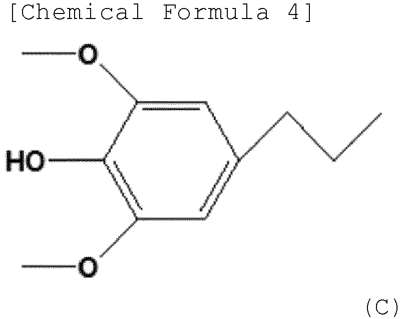

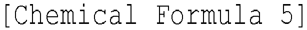

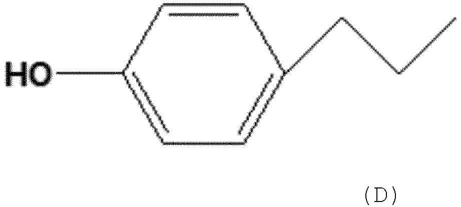

Lignin-related substances to be used for the purpose of the present invention are not subjected to any particular limitations so long as they are substances derived from lignins. As far as this specification of the present invention is concerned, lignin-related substances include substances that are regarded as model compounds of lignin such as guaiacylglycerol-β-guaiacyl ether in addition to degradation products of lignins and substances that can be obtained by processing lignins. As far as this specification is concerned, biomass is not subjected to any particular limitations so long as it contains lignins and/or lignin-related substances. For example, biomass includes natural products such as grasses and trees, substances that can be obtained by processing grasses and trees and agricultural wastes.

Biomass containing lignins and lignin-related substances (which may also be referred to as lignin-containing biomass hereinafter) can take any of the forms of, for example, solid, suspension and liquid and the forms that biomass can take vary depending on if it is preprocessed or not. For example, lignin-containing biomass can take the form of suspension obtained by crushing the biomass and adding liquid to the crushed biomass.

Lignin-containing biomass may be extracted lignins. Examples of extracted lignins include extracted liquid lignins obtained by powdering lignin-containing biomass, preparing a suspension by causing the powdered lignin-containing biomass to be suspended in a solvent that is suited for extraction of lignins so as to produce a suspension of between 0.1%W/V and 50%W/V, preferably between 1%W/V and 20%W/V, subjecting the suspension to an extraction process at temperatures between 10°C and 150°C, preferably between 20°C and 130°C, more preferably between 20°C and 80°C, for a period between several hours and several days, preferably between an hour and 6 days, and subsequently removing the solid contents from the extraction-processed solution and extracted solid lignins obtained by removing the solvent from the extracted liquid lignins and drying the evaporation residue.

Solvents that can be used for the purpose of the present invention and are suited for extraction of lignins are not subjected to any particular limiations. Examples of such solvents include water, dioxane, low molecular weight alcohols such as methanol and isopropanol, dimethyl formaldehyde and so on, of which water and dioxane are preferable.

With the above defined production method of the present invention, enzymes derived from microorganisms that belong to the genus Novosphingobium are employed in order to obtain phenyl propane-based compounds from lignin-containing biomass. Examples of such microorganisms include those belonging to the genus Novosphingobium that are known to degrade various aromatic compounds. Examples of microorganisms of the genus Novosphingobium that can be used for the production method of the present invention include Novosphingobium species MBES04 (to be also referred to as MBES04 strain hereinafter).

MBES04 strain is identified as "Novosphingobium sp. MBES04" for microorganism identification and was deposited in the Fermentation Res. Inst. Patented Microorganism Depository Center (T̅292-0818 2-5-8 Kazusakamatari, Kisarazu City, Chiba Prefecture) of the National Institute of Technology and Evaluation (NITE) on the deposition date of January 30, 2014 with deposition number of "NITE AP-01797".

With the above identified production method of the present invention, preferably two or more than two different types of enzymes derived from microorganisms of the genus Novosphingobium are employed in combination. Specific examples of such combinations include a combination of enzymes of three different types including enzymes that oxidize Cα carbon of guaiacylglycerol-β-guaiacyl ether; enzymes that reductively cleave the arylglycerol-β-aryl ether type bond at β carbon of 1-(4-hydroxy-3-methoxyphenyl)-3-hydroxy-2-(2-methoxyphenoxy)propane-1-on; and enzymes that desorb the reduction site of 3-hydroxy-1-(4-hydroxy-3-methoxyphenyl)-1-propanone having a reduction site at β carbon.

Chemical formulas (E) through (G) shown below show the chemical structures of guaiacylglycerol-β-guaiacyl ether, 1-(4-hydroxy-3-methoxyphenyl)-3-hydroxy-2-(2-methoxyphenoxy)propane-1-on and 3-hydroxy-1-(4-hydroxy-3-methoxyphenyl)-1-propanone having a reduction site at β carbon. 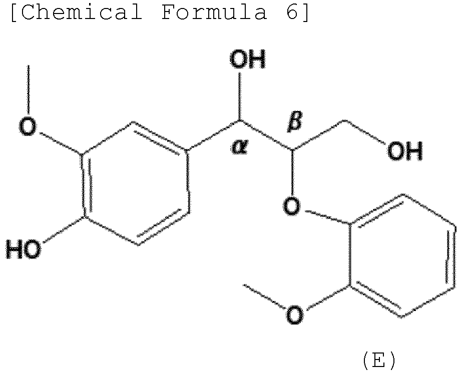 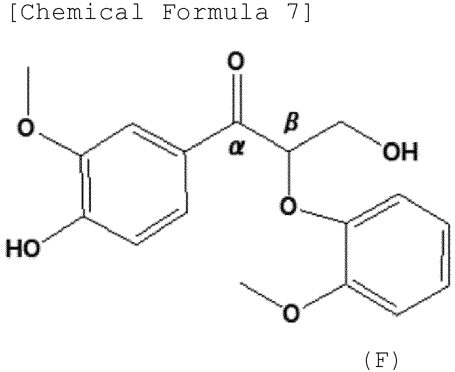 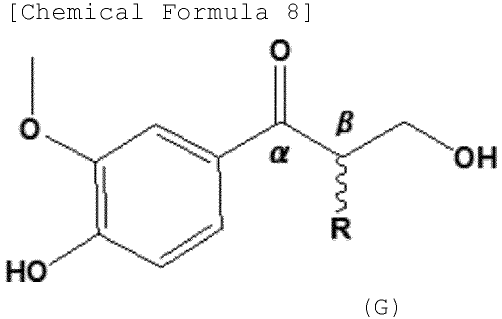 (where R indicates the reduction site thereof)

Enzymes that oxidize the Cα carbon of guaiacylglycerol-β-guaiacyl ether are those that can generate 1-(4-hydroxy-3-methoxyphenyl)-3-hydroxy-2-(2-methoxyphenoxy)propane-1-on by acting on the Cα carbon of guaiacylglycerol-β-guaiacyl ether and/or the hydroxyl group that is bonded to the α carbon and forming a carbonyl group at the site. Enzymes that reductively cleave the arylglycerol-β-aryl ether type bond at the β carbon of 1-(4-hydroxy-3-methoxyphenyl)-3-hydroxy-2-(2-methoxyphenoxy)propane-1-on are those that can generate 3-hydroxy-1-(4-hydroxy-3-methoxyphenyl)-1-propanone having a reduction site at the β carbon by acting on the arylglycerol-β-aryl ether type bond at the β carbon of 1-(4-hydroxy-3-methoxyphenyl)-3-hydroxy-2-(2-methoxyphenoxy)propane-1-on and mutually substituting the aryl ether and a reducing agent. Enzymes that desorb the reduction site of 3-hydroxy-1-(4-hydroxy-3-methoxyphenyl)-1-propanone are those that can generate 3-hydroxy-1-(4-hydroxy-3-methoxyphenyl)-1-propanone by acting on the reduction site existing at the β carbon and desorbing the reduction site.

While there are no particular limitations to the method to be used to acquire enzymes as described above, enzymes of any of the above listed types or a mixture thereof can be obtained by causing a solution of extracted enzymes or a fractionated solution thereof to act on the substrate of enzymes of the selected type or the substrates of enzymes of the selected types and checking the reduction in the quantity of the substrate or in the quantities of the substrates, whichever appropriate, and the existence of one or more than one generated substances.

There are no particular limitations to enzymes that oxidize the Cα carbon of guaiacylglycerol-β-guaiacyl ether so long as they are derived from microorganisms of the genus Novosphingobium that can exert the above identified action. Examples of such enzymes include short-chain dehydrogenase/reductase that belongs to the Rossmann-fold NAD(P)(+) binding protein super family and has Multi-domain of PRK-061904. Specific examples of short-chain dehydrogenase/reductase include C10G0069 as defined in the sequence listing with sequence number 1 and C10G0093 as defined in the sequence listing with sequence number 2. PRK06194 Multi-domain is an amino acid sequence that is often observed in the hypothetical protein; Provisional whose PSSM-Id is 180458. The E-value of PRK06194 is 1.33e-66 relative to C10G0069 and 1.35e-68 relative to C10G0093. The expression of "having Multi-domain" as used in this specification means having the amino acid sequence of Multi-domain itself and additionally having the amino acid sequence of part of Multi-domain at the side of the N end or at the side of the C end and also having an amino acid sequence approximated to that of Multi-domain.

C10G0069 and C10G0093C have physiochemical properties as described below. Namely, C10G0069 and C10G0093C oxidize the Cα carbon of guaiacylglycerol-β-guaiacyl ether in the presence of NAD+. On the other hand, these enzymes can reduce the carbonyl group residue at the α site of 1-(4-hydroxy-3-methoxyphenyl)-3-hydroxy-2-(2-methoxyphenoxy)propane-1-on in the presence of NADH and give a hydroxyl group thereto. Note, however, these enzymes do not show any activity in the presence of NAPD+ and NADPH. Therefore, with the above defined production method of the present invention, an enzyme reaction is realized in the presence of NAD. The optimum pH of C10G0069 is between 8.5 and 9.5 and the optimum temperature thereof is between 10 and 15°C. The optimum pH of C10G0093 is between 8.5 and 10.5 and the optimum temperature thereof is between 25 and 30°C. Note that the expression of the optimum pH and that of the optimum temperature as used in this specification respectively refer to the range of pH and that of temperature with which not less than 80% of the highest yield of the target product is achieved among the results obtained by the measurements in Examples that will be described hereinafter.

Since the α carbon and the β carbon of guaiacylglycerol-β-guaiacyl ether are asymmetric carbon atoms, the compound has four optical isomers of αS, βR (SR); αR, βS (RS); αR, βR (RR) and αS, βS (SS). C10G0069 specifically reacts to the two optical isomers of RS and RR of guaiacylglycerol-β-guaiacyl ether, whereas C10G0093 specifrically reacts to the remaining two optical isomers of SR and SS of the compound.

Enzymes that can be used to oxidize the Cα carbon of guaiacylglycerol-β-guaiacyl ether in the production method of the present invention are not subjected to any particular limitations so long as they have the above described physiochemical properties of C10G0069 or C10G0093. Examples of such enzymes include those that have an amino acid sequence similar to the amino acid sequence defined in the sequence listing with sequence number 1 or 2 and oxidize the Cα carbon of guaiacylglycerol-β-guaiacyl ether.

Examples of such enzymes include those whose amino acid sequences include one to several amino acid deletions, amino acid substations or amino acid additions in the amino acid sequence defined in the sequence listing with sequence number 1 or 2 and oxidize the Cα carbon of guaiacylglycerol-β-guaiacyl ether and enzymes having amino acid sequences identical with the amino acid sequence described in the sequence listing with sequence number 1 or 2 by not less than 90% and oxidize the Cα carbon of guaiacylglycerol-β-guaiacyl ether.

The range of "one to several" in the above expression of "one to several amino acid deletions, amino acid substitutions or amino acid additions in the amino acid sequence" is not subjected to any particular limitations so long as the enzymes exert an action of oxidizing the Cα carbon of guaiacylglycerol-β-guaiacyl ether within the range. However, it means, for example, 1, 2, 3, 4, 5, 6, 7, 8, 9, 10, 11, 12, 13, 14, 15, 16, 17, 18, 19 or 20, preferably 1, 2, 3, 4 or 5. The expression of "amino acid deletions" refers to lack or disappearance of amino acid residues in the sequence and the expression of "amino acid substitutions" refers to that the amino acid residues in the sequence are substituted by other amino acid residues, while the expression of "amino acid additions" refer to that fresh amino acid residues are added to the sequence.

Specific modes of "one to several amino acid deletions, amino acid substitutions or amino acid additions" include a mode where one to several existing amino acids are substituted by fresh amino acids that chemically resemble to the former amino acids. Examples of such a mode include an instance where a hydrophobic amino acid is substituted by a fresh hydrophobic amino acid and an instance where an amino acid of a given polarity is substituted by a fresh amino acid of the different polarity having the same electric charge. Such chemically similar amino acids are known to those who are skilled in the art for each and every amino acid. As specific examples, alanine, valine, isoleucine, leucine, proline, tryptophan, phenylalanine and methionine are known as nonpolar (hydrophobic) amino acids and glycine, serine, threonine, tyrosine, glutamine, asparagine and cysteine are known as polar (neutral) amino acids, whereas arginine, histidine and lysine are known as positively charged (basic) amino acids and aspartic acid and glutamic acid are known as negatively charged (acidic) amino acids.

As far as the present invention is concerned, "identity" of amino acid sequence is not subjected to any particular limitations so long as the related enzymes exert an action of oxidizing the Cα carbon of guaiacylglycerol-β-guaiacyl ether. For example, when the amino acid sequence of any of such enzymes is aligned with the amino acid sequence described in the sequence listing with sequence number 1 or 2, the sequences may be identical with each other by not less than 90%, preferably by not less than 95%, more preferably by not less than 97%, further more preferably by not less than 98%, most preferably by not less than 99%. The method to be used for determining the degree of identity of amino acid sequences is not subjected to any particular limitations. For example, the degree of identity can be determined by aligning the amino acid sequence in the sequence listing with sequence number 1 or 2 and the target amino acid sequence and computing the degree of agreement of the two sequences, using a generally known method.

The molecular weight of C10G0069 or C10G0093 is 34kDa as confirmed by means of SDS-PAGE. Note that, as far as this specification is concerned, the molecular weight refers to the molecular weight as that of E coli recombinant protein. Thus, the molecular weight of the enzyme that has an amino acid sequence that resembles to the amino acid sequence described in the sequence listing with sequence number 1 or 2 and oxidizes the Cα carbon of guaiacylglycerol-β-guaiacyl ether is similar to the molecular weight of C10G0069 or C10G0093 and preferably between 30 and 40 kDa, more preferably between 32 and 36 kDa, further more preferably between 33 and 35kDa, most preferably close to 34 kDa as confirmed by means of SDS-PAGE.

For the purpose of the present invention, any method can be used to obtain enzymes that oxidize the Cα carbon of guaiacylglycerol-β-guaiacyl ether without limitations. For example, a screening method of looking into the enzyme activity in the culture supernatant obtained by cultivating microorganisms of the genus Novosphingobium such as those of the genus Novosphingobium living in well rotten sunken wood of coniferous trees in deep sea (not less than 200 m below from the sea level), typically using the enzyme action, the substrate (optical isomer) specificity, the physiochemical properties, the molecular weight and so on of C10G0069 or C10G0093 as indexes, may be employed. Alternatively, such enzymes may be synthesized by a physiochemical means, by referring to the descriptions in the sequence listing with sequence number 1 or 2 or prepared from genes having a base sequence for encoding the amino acid sequence described in the sequence listing with sequence number 1 or 2 by means of genetic engineering techniques. Note that the genes having a base sequence for encoding the amino acid sequence described in the sequence listing with sequence number 1 or 2 are described respectively in the sequence listing with sequence number 7 or 8, whichever appropriate.

For the purpose of the present invention, there are no particular limitations to enzymes that can reductively cleave the arylglycerol-β-aryl ether type bond at the β carbon of 1-(4-benzyloxy-3-methoxyphenyl)-3-hydroxy-2-(2-methoxyphenoxy)propane-1-on so long as they are derived from microorganisms of the genus Novosphingobium capable of exerting the above described action. Examples of such enzymes include glutathione S-transferase that has Multi-domain of PRK15113 and maiA at the side of Gst and the N end. Specific examples of glutathione S-transferase include C10G0076 defined in the sequence listing with sequence number 3 or C10G0077 defined in the sequence listing with sequence number 4. Gst is an amino acid sequence that is often found in glutathione S-transferase whose PSSM-Id is 2223698. PRK15113 is an amino acid sequence that is often found in glutathione S-transferase; Provisional whose PSSM-Id is 185068. maiA is an amino acid sequence that is often found in malaylacetoacetate isomerase whose PSSM-Id is 233333. The E-value of Gst is 4.21e-38 relative to C10G0076 and 6.35e-11 relative to C10G0077. The E-value of PRK15113 is 3.10e-16 relative to C10G0076 and 4.58e-03 relative to C10G0077. The E-value of maiA is 8.01e-17 relative to C10G0076 and 1.01e-05 relative to C10G0077. C10G0076 acts on the βS optical isomer of 1-(4-hydroxy-3-methoxyphenyl)-3-hydroxy-2-(2-methoxyphenoxy)propane-1-on, whereas C10G0077 acts on the βR optical isomer of 1-(4-hydroxy-3-methoxyphenyl)-3-hydroxy-2-(2-methoxyphenoxy)propane-1-on. Note that the genes having the base sequence that encodes the amino acid sequences described in the sequence listing with sequence numbers 3 and 4 are defined respectively in the sequence listings with sequence numbers 9 and 10.

There are no particular limitations to enzymes that can desorb the reduction site of 3-hydroxy-1-(4-hydroxy-3-methoxyphenyl)-1-propanone having a reduction site at the β carbon so long as they are derived from microorganisms of the genus Novosphingobium and can exert the above identified action. Examples of such enzymes include glutathione S-transferase that has Multi-domain of PRK10387 and maiA at the side of Gst and the N end and glutathione S-transferase that has Multi-domain of Gst, PRK 11752, PRK15113 and maiA. Specific examples of glutathione S-transferase that has Multi-domain of PRK 10387 and maiA at the side of Gst and the N end include C10G0078 as defined in the sequence listing with sequence number 5. Specific examples of glutathione S-transferase that has Multi-domain of Gst, PRK 11752, PRK15113 and maiA include C10G0075 defined in the sequence listing with sequence number 6. PRK10387 is an amino acid sequence that is often found in glutaredoxin2; Provisional whose PSSM-Id is 236679. PRK11752 is an amino acid sequence that is often found in putative S-transfarase; Provisional whose PSSM-Id is 183298. The E-value of Gst is 2.26e-14 relative to C10G0078 and 5.08e-44 relative to C10G0075. The E-value of maiA is 9.25e-03 relative to C10G0078 and 4.73e-09 relative to C10G0075. The E-value of PRK10387 is 0.02 relative to C10G0078. The E-value of PRK11752 is 5.41e-52 relative to C10G0075. The E-value of PRK15113 is 1.11e-12 relative to C10G0075. C10G0078 acts on substances derived from the βS optical isomer of 1-(4-hydroxy-3-methoxyphenyl)-3-hydroxy-2-(2-methoxyphenoxy)propane-1-on and C10G0075 acts on substances derived from the βS optical isomer and the βR optical isomer of 1-(4-hydroxy-3-methoxyphenyl)-3-hydroxy-2-(2-methoxyphenoxy)propane-1-on. Note that the genes having the base sequences that encode the amino acid sequences described in the sequence listings with sequence numbers 5 and 6 are defined respectively in the sequence listings with sequence numbers 11 and 12.

When reduction type glutathione is added so as to coexist in the reaction system, C10G0076 and C10G0077, which are glutathione S-transferases, catalyze the activity of cleavage of the β-O-4 type bond due to the addition of glutathione but they do not show any activity when reduction type glutathione does not coexist. Similarly, C10G0078 and C10G0075 catalyze the activity of desorbing the glutathione added to the β carbon when reduction type glutathione coexists but they do not show any activity when reduction type glutathione does not coexist. Therefore, with the production method of the present invention, enzyme reactions are realized in the presence of reduction type glutathione.

Phenyl propane-based compounds can be obtained by causing enzymes derived from microorganisms of the genus Novosphingobium to act on lignin-containing biomass. The expression of "causing enzymes to act on" as used herein means exerting the action of oxidizing the Cα carbon of guaiacylglycerol-β-guaiacyl ether, exerting the action of reductively cleaving the arylglycerol-β-aryl ether type bond at the β carbon of 1-(4-hydroxy-3-methoxyphenyl)-3-hydroxy-2-(2-methoxyphenoxy)propane-1-on and exerting the action of desorbing the reduction site of 3-hydroxy-1-(4-hydroxy-3-methoxyphenyl)-1-propanone having the reduction site at the β carbon.

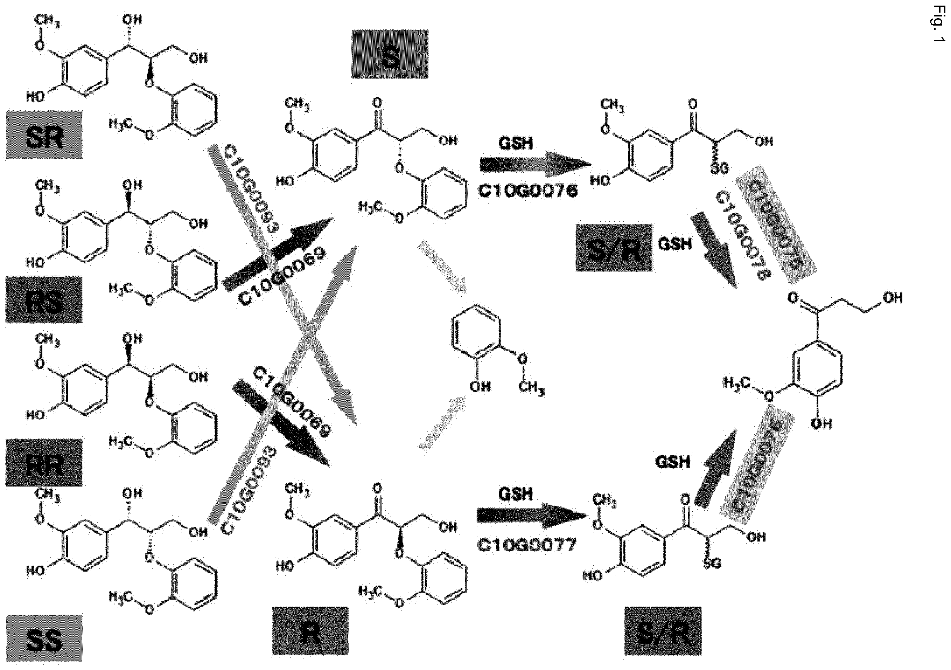

As described above, C10G0069, C10G0093, C10G0076, C10G0077, C10G0078 and C10G0075 are selectively employed as a function of the optical isomers of the compound that operates as substrate. FIG. 1 schematically illustrates the relationship between these enzymes and the optical isomers on which they act. (1) through (21) below shows so many conceivable combinations of these enzymes.
(1) C10G0069, C10G0076 and C10G0078
(2) C10G0069, C10G0076 and C10G0075
(3) C10G0069, C10G0076, C10G0078 and C10G0075
(4) C10G0069, C10G0077 and C10G0075
(5) C10G0093, C10G0076 and C10G0078
(6) C10G0093, C10G0076 and C10G0075
(7) C10G0093, C10G0076, C10G0078 and C10G0075
(8) C10G0093, C10G0077 and C10G0075
(9) C10G0069, C10G0076, C10G0077 and C10G0078
(10) C10G0069, C10G0076, C10G0077 and C10G0075
(11) C10G0069, C10G0076, C10G0077, C10G0078 and C10G0075
(12) C10G0093, C10G0076, C10G0077 and C10G0078
(13) C10G0093, C10G0076, C10G0077 and C10G0075
(14) C10G0093, C10G0076, C10G0077, C10G0078 and C10G0075
(15) C10G0069, C10G0093, C10G0076 and C10G0078
(16) C10G0069, C10G0093, C10G0076 and C10G0075
(17) C10G0069, C10G0093, C10G0076, C10G0078 and C10G0075
(18) C10G0069, C10G0093, C10G0077 and C10G0075
(19) C10G0069, C10G0093, C10G0076, C10G0077 and C10G0078
(20) C10G0069, C10G0093, C10G0076, C10G0077 and C10G0075
(21) C10G0069, C10G0093, C10G0076, C10G0077, C10G0078 and C10G0075

When, for example, a mixture of these enzymes is employed to cause them to act on lignin-containing biomass, it is preferable to select pH and temperature at which C10G0069, C10G0093, C10G0076, C10G0077, C10G0078 and C10G0075 are active in the presence of NAD for activating C10G0069 and C10G0093 and also in the presence of reduction type glutathione for activating C10G0076 and C10G0077. These enzymes may be employed separately and NAD and reduction type glutathione may be added at different respective timings. For example, after causing C10G0069 and C10G0093 to act on lignin-containing biomass in the presence of NAD, C10G0076, C10G0077, C10G0078 and C10G0075 may be sequentially caused to act on the lignin-containing biomass in the presence of reduction type glutathione. A buffer agent is preferably added to the biochemical reaction system in order to suppress pH fluctuations each time when one or more than one of the enzymes exert their actions.

At the time when one or more than one of the enzymes are caused to act, there are no limitations to the concentration and the quantity of each of the components of the biochemical reaction system including the lignins, the lignin-related substances, the buffer agent, the enzymes, NAD and the reduction type glutathione and hence the concentrations and the quantities of the components may appropriately be selected. Additionally, the mixture of these components may be agitated or otherwise shaken in order to raise the contact frequency between the lignins and/or the lignin-related substances and the enzymes. There are no particular limitations to the acting time of the enzymes so long as generation of phenyl propane-based compounds is observed during the acting time. For example, the acting time may be between several hour and several days. It may appropriately be selected as a function of the titer of each of the enzymes. More specifically, the acting time is typically between an hour and about 36 hours or more.

While the phenyl propane-based compounds that are generated in the biochemical reaction system can be put to use without subjecting them to one or more than one additional special treatment processes, they may be refined by using one or more than one known ordinary aromatic compounds refining techniques such as a solid phase extraction method or a chromatogram method after the end of the reactions in the biochemical reaction system. Furthermore, a solid reaction product can be obtained by removing the solvent and drying the reaction product.

With the production method of the present invention, one or more than one different steps and/or operations may be added before, during or after the end of the reactions in the biochemical reaction system.

A specific exemplar mode of carrying out the production method of the present invention will be described below.

Lignin-containing biomass is suspended in a solvent that is suited for dissolving lignins such as dioxane and water and the suspension is held to temperatures between 20 and 80°C for a period between several hours and several days. After the temperature keeping period, the solid components are removed from the suspension to obtain solution of extracted lignins. Then, the extracted lignins are dried and solidified and the obtained dried and solidified product is dissolved in an organic solvent or a water-containing organic solvent such as ethyl acetate or DMF to obtain extracted lignins. Then a reaction solution containing the extracted lignins, enzymes that oxidize the Cα carbon of guaiacylglycerol-β-guaiacyl ether, enzymes that reductively cleave the arylglycerol-β-aryl ether type bonds at the β carbon of 1-(4-hydroxy-3-methoxyphenyl)-3-hydroxy-2-(2-methoxyphenoxy)propane-1-on, enzymes that desorb the reduction site of 3-hydroxy-1-(4-hydroxy-3-methoxyphenyl)-1-propanone having the reduction site at the β carbon thereof, a buffer agent, NAD salt and reduction type glutathione is prepared and the components are caused to react at pH between 7 and 10, at temperature between 20 and 40°C for a duration of time between several hours and tens of several hours. After the reaction, the reaction product solution is refined by a solid phase extraction method and dried in the presence of inert gas to obtain solid phenyl propane-based compounds.

The phenyl propane-based compounds obtained by the production method of the present invention can be utilized as starting materials or intermediates for producing resins, adhesive agents, resist materials and drugs. More specifically, when 3-hydroxy-1-(4-hydroxy-3-methoxyphenyl)-1-propanone is obtained by the production method of the present invention, the compound can be transformed into coniferyl alcohol and so on that are industrially useful as starting materials of drugs, perfumes, food materials and so on by way of 1-(4-hydroxy-3-methoxyphenyl)-1,3-propanediol.

In another aspect of the present invention, there is disclosed a method for producing carbonyl phenyl-based compounds comprising a step of obtaining carbonyl phenyl-based compounds by causing enzymes defined in (1) below and derived from microorganisms of the genus Novosphingobium to act on biomass containing lignins and/or lignin-related substances in the presence of NAD.
(1) Short-chain dehydrogenase/reductase that belongs to the Rossmann-fold NAD(P)(+) binding protein super family, has Multi-domain of PRK06194 and oxidizes the Cα carbon of guaiacylglycerol-β-guaiacyl ether

For example, the HPLC analysis conditions of 1-(4-hydroxy-3-methoxyphenyl)-3-hydroxy-2-(2-methoxyphenoxy)propane-1-on can be adopted for the method of confirming the production of carbonyl phenyl-based compounds. More specifically, 1-(4-hydroxy-3-methoxyphenyl)-3-hydroxy-2-(2-methoxyphenoxy)propane-1-on can qualitatively and quantitatively be analyzed, for example, by means of reverse phase HPLC. The conditions to be met for reverse phase HPLC are, for example, that an Octa Decyl Silyl group-modified silica gel column (ODS column) is to be employed along with eluent A (2mM ammonium acetate 0.05%V/V formic acid) and eluent B (100%V/V methanol), that a column temperature of 40°C and a flow rate of 1.2 ml/min are to be set and that a mixture solution of eluent A 90%V/V and eluent B 10%V/V is to be fed for a minute and subsequently eluent B is to be fed at a gradient of 10%V/V to 95%V/V for 7 minutes. 3-hydroxy-1-(4-hydroxy-3-methoxyphenyl)-1-propanone can be detected under these conditions as a peak of retention time of about 6. 5 minutes by means of a UV detector (270 nm). It can be quantified by means of a calibration curve method, an internal standard method or the like when standard 1-(4-hydroxy-3-methoxyphenyl)-3-hydroxy-2-(2-methoxyphenoxy)propane-1-on is employed.

In a preferable mode, the enzyme described in (1) above is short-chain dehydrogenase/reductase C10G0069 as defined in the sequence listing with sequence number 1 or short-chain dehydrogenase/reductase C10G0093 as defined in the sequence listing with sequence number 2.

In still another aspect of the present invention, there is provided short-chain dehydrogenase/reductase that is derived from microorganisms of the genus Novosphingobium, belongs to the Rossmann-fold NAD(P)(+) binding protein super family, has Multi-domain of PRK06194 and oxidizes the Cα carbon of guaiacylglycerol-β-guaiacyl ether.

In a preferable mode, the enzyme of the present invention preferably is short-chain dehydrogenase/reductase that has a molecular weight of 34 kDa, which is confirmable by means of SDS-PAGE, and whose optimum pH and optimum temperature are respectively between 8.5 and 9.5 and between 10 and 15°C or short-chain dehydrogenase/reductase that has a molecular weight of 34 kDa, which is confirmable by means of SDS-PAGE, and whose optimum pH and optimum temperature are respectively between 8.5 and 10.5 and between 25 and 30°C.

The enzyme of the present invention as defined above is short-chain dehydrogenase/reductase C10G0069 having an amino acid sequence as defined in the sequence listing with sequence number 1 or short-chain dehydrogenase/reductase C10G0093 having an amino acid sequence as defined in the sequence listing with sequence number 2.

Now, the present invention will be described in greater detail by way of examples. Note, however, that the present invention is by no means limited by the examples that are described below. In other words, the present invention can be carried out in various different modes so long as the present invention can dissolve the above identified problem to be solved in any of such modes.

### EXAMPLES

### [Example 1. Detection of Genes in MBES04 Strain]

Novosphingobium MBES04 strain was inoculated in an LB culture medium (available from Becton Dickinson), to which 5 mM magnesium sulfate had been added, and subjected to shaking culture at 30°C and 120rpm for 24 hours. Bacterial cells of MBES04 strain were obtained from the produced culture solution by means of centrifugal separation conducted at 8,000 rpm for 5 minutes.

The total DNA was extracted from the obtained bacterial cells by means of a NucleoSpin PlantII Midi Kit (available from TAKARA BIO). A 8kb-long mate pair library was prepared by using the extracted DNA and the DNA sequence was analyzed by means of a genome sequencer GS FLX System (available from Roche Diagnostics). As a result of analyzing the DNA sequence, a DNA sequence having sequence data including the number of reads of 142,389, the average length of 441.66 bp and the total number of bases of 62,888,162 was obtained.

A base sequence assembly operation was executed for the obtained base sequence by means of Analysis Software Newbler 2.6 (available from Roche Diagnostics). As a result, 783 contigs having an average length of 6434.5 bp were formed. Subsequently, a scaffold was generated on the basis the mate pair information obtained by scrutinizing the sequence of each of the contigs from the information at the opposite ends of the sequence of the 8kB mate pair library to obtain 37 super contigs consisting of a total number of bases of 5,596,306. Furthermore, part of the genome sequence was re-analyzed by means of Sanger's method to obtain 39 super contigs. Then, the gene region for encoding in the total DNA sequence included in the 39 super contigs, namely the region that corresponds to the open reading frame for encoding proteins was presumptively determined by using the GeneMarkS method (see Non-Patent Document 4).

### [Example 2. Search for Lignin Degrading Enzymes]

LigD, LigL, LigN and LigO, which are enzymes that catalyze oxidation of the hydroxyl group bonded to the Cα carbon of guaiacylglycerol-β-guaiacyl ether (Compound I), which is the first step reaction of the process where the SYK-6 strain metabolizes Compound I or lignin model dimer compound (see Non-Patent Documents 5 and 6), are known along with gene ligD (YP_004833998), gene ligL (AB491221), gene ligN (AB491222) and gene ligO (YP_004836720) that respectively encode those enzymes.

Thus, a BLAST homology search (blast 2. 2.26; National Center for Biotechnology Information) was conducted to search for genes that show homology relative to gene ligD, gene ligL, gene ligN and gene ligO, which were derived from SYK-6 strain, on the basis of threshold: Coverage 50%<, Identity 25%<, Similarity 50%< and E value <e5, using the genome sequence information on the MBES04 strain, which is obtained in Example 1, as query for the search.

As a result, a total of six genes including gene c01g1162, gene c01g1324, gene c10g0069, gene c10g0080, gene c10g0093 and gene c10g0094 that have sequences annotated with Short-chain dehydrogenase/reductase were found from the genome sequence information on the MBES04 strain.

Table 1 shows information relating to the homology of the above described MBES04 strain-derived six genes and SYK-derived genes of ligD, ligL, ligN and ligO.

**[Table 1]**

| #Query | Query_ Length (bp) | Subject | Subject_ length (bp) | Score | E-value | Query_ coverage (%) | Subject_ coverrage (%) | Identity (%) | Similarity (%) | Gap |
|---|---|---|---|---|---|---|---|---|---|---|
| c01g1162 | 246 | ligO | 297 | 107 | 1.00E-27 | 78 | 65 | 33 | 56 | 4/196 |
| | 246 | | 289 | 103 | 1.00E-26 | 77 | 67 | 14 | 52 | 4/194 |
| c01g1324 | 276 | ligL | 289 | 79.7 | 3.00E-19 | 65 | 65 | 31 | 51 | 10/189 |
| | 276 | ligO | 297 | 75.5 | 5.00E-18 | 64 | 61 | 31 | 51 | 8/184 |
| c10g0069 | 303 | ligO | 297 | 318 | 3.00E-91 | 94 | 95 | 57 | 70 | 6/288 |
| | 303 | ligD | 305 | 231 | 5.00E-65 | 94 | 92 | 44 | 59 | 7/287 |
| | 303 | ligL | 289 | 196 | 2.00E-54 | 96 | 99 | 39 | 57 | 12/296 |
| | 303 | ligN | 311 | 178 | 4.00E-49 | 94 | 91 | 38 | 54 | 14/292 |
| c10g0080 | 309 | ligO | 297 | 194 | 6.00E-54 | 91 | 96 | 37 | 58 | 10/291 |
| | 309 | ligD | 305 | 170 | 1.00E-46 | 91 | 93 | 34 | 56 | 11/290 |
| | 309 | ligL | 289 | 169 | 2.00E-46 | 86 | 92 | 36 | 57 | 7/271 |
| | 309 | ligN | 311 | 127 | 2.00E-33 | 88 | 89 | 35 | 51 | 9/280 |
| c10g0093 | 311 | ligO | 297 | 216 | 2.00E-60 | 85 | 90 | 45 | 59 | 13/275 |
| | 311 | ligN | 311 | 199 | 3.00E-55 | 92 | 95 | 45 | 57 | 23/304 |
| | 311 | ligL | 289 | 194 | 7.00E-54 | 88 | 95 | 40 | 57 | 10/280 |
| | 311 | ligD | 305 | 184 | 6.00E-51 | 85 | 88 | 38 | 57 | 2/269 |
| c10g0094 | 299 | ligO | 297 | 217 | 8.00E-61 | 93 | 95 | 42 | 60 | 4/285 |
| | 299 | ligD | 305 | 202 | 3.00E-56 | 91 | 90 | 38 | 57 | 3/276 |
| | 299 | ligL | 289 | 185 | 3.00E-51 | 91 | 96 | 37 | 56 | 6/279 |
| | 299 | ligN | 311 | 146 | 3.00E-39 | 83 | 83 | 36 | 56 | 10/261 |

A homology search (BLASTn, http://blast.ncbi.nlm.nih.gov/Blast.cgi?PROGRAM=blastn&PAG GE_TYPE=BlastSearch&LINK_LOC=blasthome, Coverage 50%<) was conducted, using the base sequences of the MBES04 strain-derived six genes of gene c01g1162, gene c01g1324, gene c10g0069, gene c10g0080, gene c10g0093 and gene c10g0094 as query for the search. As a result, it was found that the genes that were hit as those showing the highest degree of sequence agreement are as follows. With regard to gene c01g1162, 71% agreement was for the gene that presumably encodes short-chain dehydrogenase/reductase located at the 4935562th through 4936300th positions on the genome (CP001656.1) of Paenibacillus sp. JDR-2 strain; with regard to gene c01g1324, 75% agreement was for the gene that presumably encodes short-chain dehydrogenase/reductase located at the 1154509th through 1155339th positions on the genome (FR856862.1) of Novosphingobium sp. PP1Y strain; with regard to gene c01g0069, 78% agreement was for the gene that presumably encodes short-chain dehydrogenase/reductase located at the 1241135th through 1242028th positions on the genome (FR856862.1) of Novosphingobium sp. PP1Y strain; with regard to gene c01g0080, 76% agreement was for the gene that presumably encodes short-chain dehydrogenase/reductase located at the 1251645th through 1252546th positions on the genome (FR856862.1) of Novosphingobium sp. PP1Y strain; with regard to gene c01g0093, 80% agreement was for the gene that presumably encodes short-chain dehydrogenase/reductase located at the 843288th through 844180th positions on the genome (CP000248.1) of Novosphingobium aromaticivorans DSM 12444 strain; with regard to gene c01g0094, 77% agreement was for the gene that presumably encodes short-chain dehydrogenase/reductase located at the 1266103th through 1266974th positions on the genome (FR856862.1) of Novosphingobium sp. PP1Y strain.

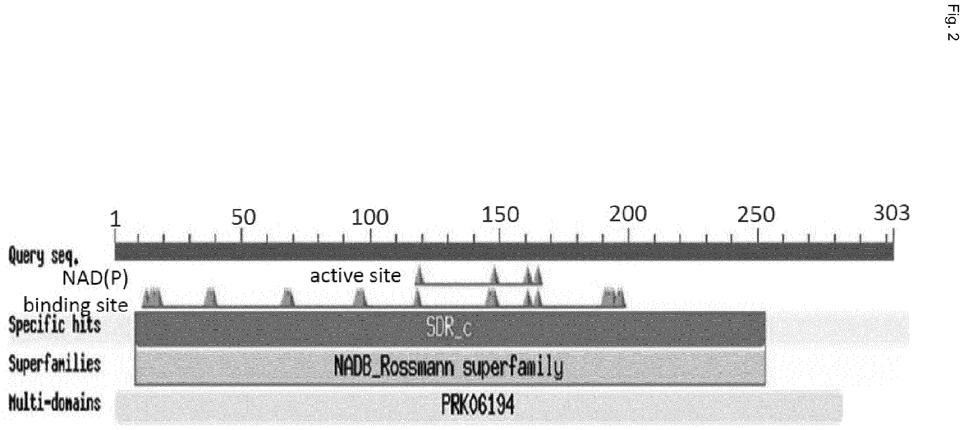

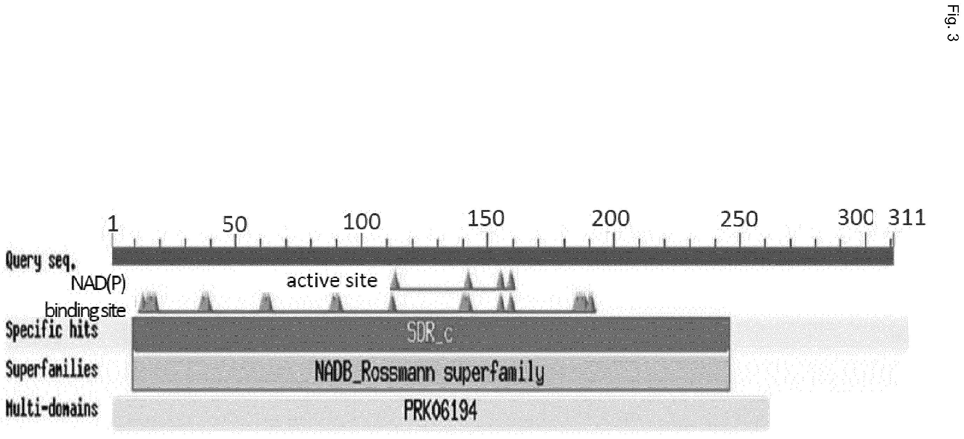

A DELTA-BLAST search (National Center for Biotechnology Information, Domain Enhanced Lookup Time Accelerated BLAST)(http://blast.ncbi.nim.nih.gov/Blast.cgi?PAGE_TYPE=B lastSearch&PROGRAM=blast&BLAST_PROGRAMS=deltaBlast) was conducted, using the amino acid sequences that gene c10g0069 and gene c10g0093 encode out of the above six genes. As a result, it was found that all the proteins that the above two genes encode are short-chain dehydrogenase/reductase that belongs to the Rossmann-fold NAD(P)(+) binding protein super family and has Multi-domain of PRK06194 (see FIGS. 2 and 3).

On the other hand, gene ligF (YP_004833997)(see Non-Patent Document 7) and gene ligE (YP_004833998)(see Non-Patent Document 8) are known as enzymes that catalyze the second step reaction of the process where the SYK-6 strain metabolizes Compound I or the reaction of cleaving the ether bond at β-O-4 position of 1-(4-hydroxy-3-methoxyphenyl)-3-hydroxy-2-(2-methoxyphenoxy)propane-1-on (compound II). Additionally, gene ligG (YP_004833999)(see Non-Patent Document 9) is known as enzyme that catalyzes the third step reaction of removing glutathione from the glutathione-containing intermediate produced from the above described second step reaction.

Thus, a BLAST homology search was conducted to search for genes that show homology relative to SYK-6 strain-derived genes of gene ligE, gene ligF and gene ligG on the basis of threshold: Coverage 50%<, Identity 25%<m Similarity 50%< and E-value <e5, using the genome sequence information on the MBES04 strain detected in Example 1 as query for the search. As a result, it was found that gene c10g0076 shows 68% identity relative to ligF, gene c10g0077 shows 80% identity relative to ligE and gene c10g0078 shows 71% identity relative to ligG.

A BLASTn homology search (Coverage 50%<) was conducted, using the base sequences of the three genes of gene c10g0076, gene c10g0077 and gene c10g0078, which are derived from MBES04 strain, as query for the search. As a result, it was found that the genes that were hit as those showing the highest degree of sequence agreement are as follows. With regard to gene c10g0076, 82% agreement was for the gene that presumably encodes glutathione S-transferase-like protein located at the 1247655th through 1248381th positions on the genome (FR856862.1) of Novosphingobium sp. PP1Y strain; with regard to gene c10g0078, 80% agreement was for the gene that presumably encodes glutathione S-transferase-like protein located at the 1248451th through 1249198th positions on the genome (FR856862.1) of Novosphingobium sp. PP1Y strain; with regard to gene c10g0079, 78% agreement was for the gene that presumably encodes glutathione S-transferase-like protein located at the 1249325th through 1250064th positions on the genome (FR856862.1) of Novosphingobium sp. PP1Y strain.

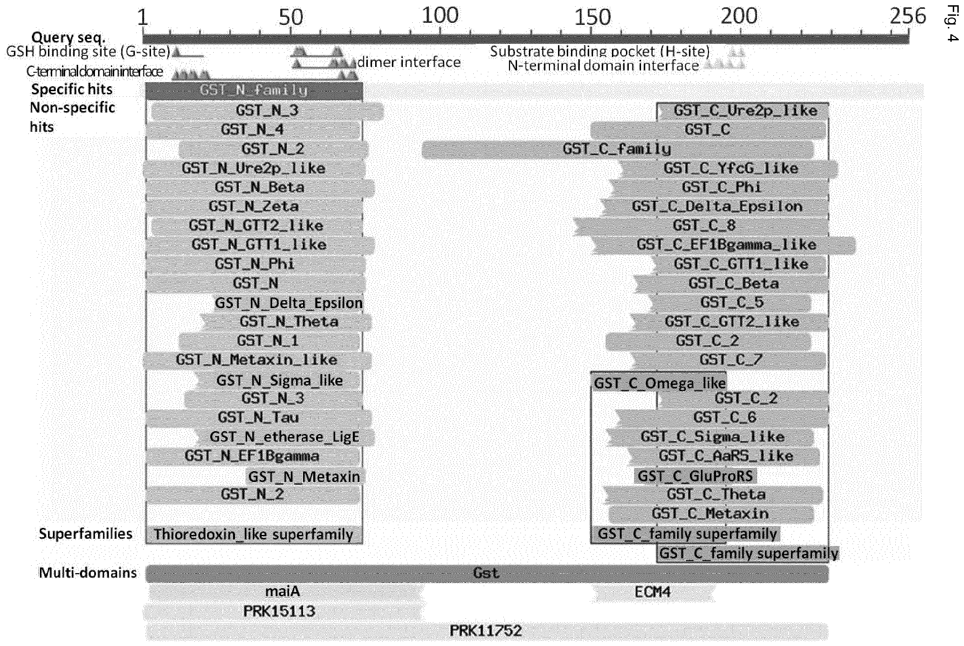

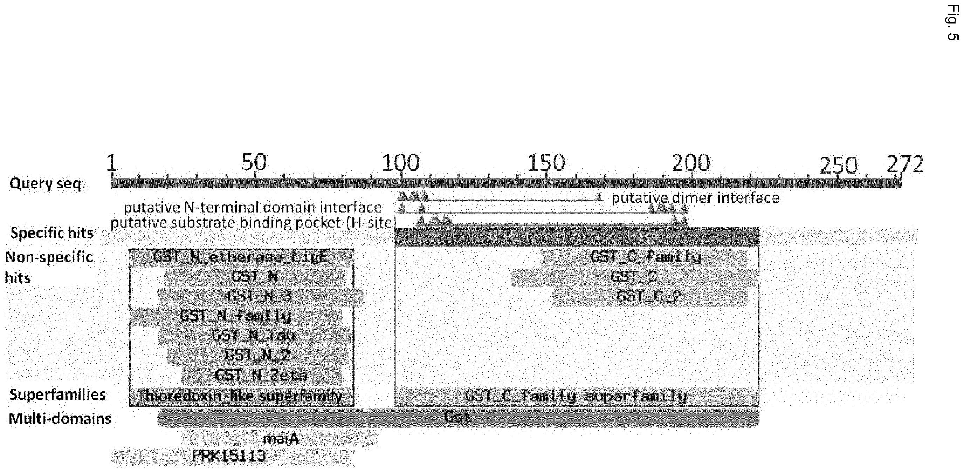

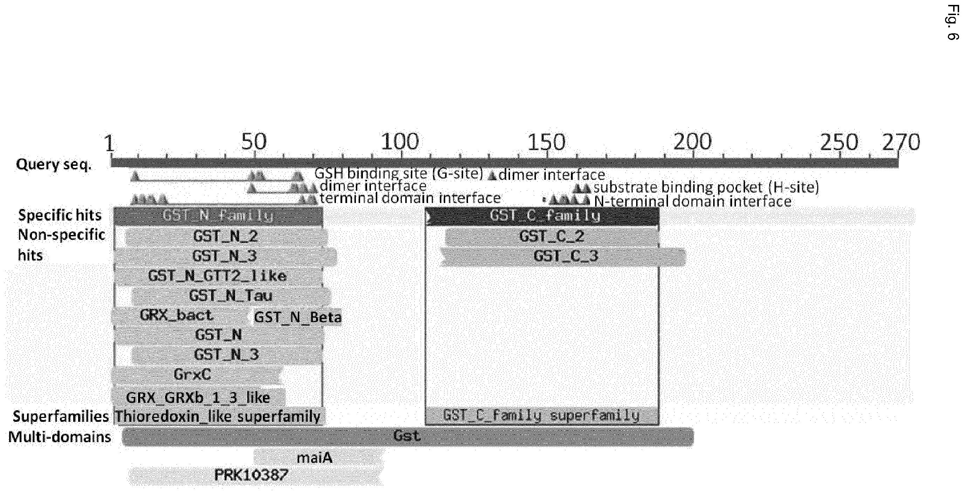

Additionally, a DELTA-BLAST search (National Center for Biotechnology Information, Domain Enhanced Lookup Time Accelerated BLAST)(http://blast.ncbi.nlm.nih.gov/Blast.cgi?PAGE_TYPE=B lastSearch&PROGRAM=blast&BLAST_PROGRAMS=deltaBlast) was conducted, using the amino acid sequences that gene c10g0076, gene c10g0077 and gene c10g0078 encode (see FIGS. 4 through 6). As a result, it was suggested by the search that all the protein sequences that the above three genes encode have the Gst (COG0625) sequence that is Multi-domain stored in the protein that is presumed to be glutathione S-transferase. Still additionally, it was found that the amino acid sequences that gene c10g0076 and gene c10g0077 encode have Multi-domain of PRK15113 and maiA at the side of the N end. Furthermore, the amino acid sequence that gene c10g0078 encodes has Multi-domain of PRK10387 and maiA at the side of the N end.

It was found that gene c10g0076, gene c10g0077 and gene c10g0078 are close to each other. Additionally, gene c10g0075 that is contiguous to gene c10g0076 encodes the protein that is presumed to be glutathione S-transferase in the direction opposite to the direction in which gene c10g0076, gene c10g0077 and gene c10g0078 encode. A BLASTn search (Coverage 50%<)was conducted, using the base sequence of gene c10g0075 as query for the search, to find that the gene sequence that was hit with the highest degree of agreement, or 79% agreement, was the gene that presumably encodes glutathione S-transferase located at the 1246742th through 1247371th positions on the genome (FR856862.1) of Novosphingobium sp. PP1Y strain. Additionally, a DELTA-BLAST search was conducted, using the amino acid sequence that gene c10g0075 encodes as query for the search. It was suggested by the search that the protein that c10g0075 gene encodes has the Multi-domain sequence stored in proteins such as Gst, PRK11752, PRK15113 and maiA that are presumed to be glutathione S-transferase.

### [Example 3. Enzyme Production Utilizing Transformants]

In order to confirm that the proteins that are derived from MBES04 strain, which is annotated with Short-chain dehydrogenase/reductase, and to be encoded by six genes of gene c01g1162, gene c01g1324, gene c10g0069, gene c10g0080, gene c10g0093 and gene c10g0094 encode, and also the proteins that are to be encoded by four genes of gene c10g0075, gene c10g0076, gene c10g0077 and gene c10g0078 are involved in the metabolism of Compound I, transformants that operate for recombinant production of the proteins to be encoded by these genes were prepared, using E coli as host.

The proteins that are to be encoded respectively by the six genes of gene c01g1162, gene c01g1324, gene c10g0069, gene c10g0080, gene c10g0093 and gene c10g0094 are expressed as C01G1162, C01G1324, C10G0069, C10G0080, C10G0093 and C10G0094, while the proteins that are to be encoded respectively by the four genes of gene c10g0075, gene c10g0076, gene c10g0077 and gene c10g0078 are expressed as C10G0075, C10G0076, C10G0077 and C10G0078.

A PCR reaction was made to take place, using plasmid pRSETA (available from Invitrogen) as template and also using primers A and B shown in Table 2. On the other hand, another PCR reaction was made to take place, using the primer set (C and D, E and F, G and H, I and J, K and L, M and N, O and P, Q and R, S and T and U and V) shown in Table 2 and also using the genome DNA of MBES04 strain as template under the conditions shown below to obtain cDNA by amplification of the gene fragments listed above.

**[Table 2]**

| **Name of the primer** | **Amplified fragments (gene)** | **Sequence** | **Sequence Number** |
|---|---|---|---|
| A | vector (pRSETA) | TCTCGAGCTCGGATCC | 13 |
| B | vector (pRSETA) | CTGGTACCATGGAATTCG | 14 |
| C | c01g1162 | GGATCCGAGCTCGAGATGATCAAGGGTATCGAAGG | 15 |
| D | c01g1162 | CGAATTCCATGGTACCAGTCAGAACTCCTGTGCGG | 16 |
| E | c01g1324 | GGATCCGAGCTCGAGATGACGAACTGGCTTATCAC | 17 |
| F | c01g1324 | CGAATTCCATGGTACCAGTCAGACCTCGGCGAAG | 18 |
| G | c10g0069 | GGATCCGAGCTCGAGATGACACAGGTAAAGGGACG | 19 |
| H | c10g0069 | CGAATTCCATGGTACCAGTCATGCCGTCTTTTCCTC | 20 |
| I | c10g0080 | GGATCCGAGCTCGAGATGGGAGAGACGACAAAAC | 21 |
| J | c10g0080 | CGAATTCCATGGTACCAGTCAGGTGAGGTCGGC | 22 |
| K | c10g0093 | GGATCCGAGCTCGAGATGCAGGATCTACCGGG | 23 |
| L | c10g0093 | CGAATTCCATGGTACCGCAAGCTGTGTCATGC | 24 |
| M | c10g0094 | GGATCCGAGCTCGAGATGACGGGCGGGG | 25 |
| N | c10g0094 | CGAATTCCATGGTACCAGTCAGAGCGCGTTGGC | 26 |
| O | c10g0075 | GGATCCGAGCTCGAGATGCTGGAACTGTGGACTTC | 27 |
| P | c10g0075 | CGAATTCCATGGTACCAGGTAGGTGTGCTCATCGTTCA | 28 |
| Q | c10g0076 | GGATCCGAGCTCGAGATGTTGACGCTGTACAGCTTTG | 29 |
| R | c10g0076 | CGAATTCCATGGTACCAGCTCCTCAGGCCTGTGC | 30 |
| S | c10g0077 | GGATCCGAGCTCGAGATGGCCAAGGACAACC | 31 |
| T | c10g0077 | CGAATTCCATGGTACCAGTCAGCTCGCCGTAGC | 32 |
| U | c10g0078 | GGATCCGAGCTCGAGATGGCATGGGACGATG | 33 |
| V | c10g0078 | CGAATTCCATGGTACCAGGATGACGGTGTGCTTCAC | 34 |

### PCR conditions

1 × PCR buffer (containing MgCl₂) 200 µm dNTPs, 0.6 µm 27f, 0.6 µm 1525r, 1.4 U of LA Taq DNA Polymerase (available from TAKARA BIO), thermal cycler temperature condition 97°C 2 minutes (97°C 30 seconds, 60°C 1 minute, 72°C 90 seconds) × 30 cycles, 72°C 5 minutes

A type of DNA fragment amplified by a PCR reaction using a primer set shown in Table 2 and pRSETA as template and another type of DNA fragment obtained by using the genome DNA of MBES04 strain as template were mixed with each other and subsequently coupled with each other by using In fusion HD cloning kit (available from TAKARA BIO) to obtain a recombinant plasmid. The obtained recombinant plasmid was transformed by introducing it into E coli (Stellar Competent cell) for each gene.

Plasmid was prepared from the obtained transformant and that the base sequence of a coupled fragment on the plasmid and the gene sequence of the MBES04 strain on the genome completely agree with each other was confirmed by a sequence analysis. For the base sequence analysis, BigDye (registered trade mark) Terminator v3.1 Cycle Sequencing Kit, v3.1 (available from Applied Biosystems) and ABI 3730 XL DNA Analyzer (available from Applied Biosystems) were employed. Subsequently, BL21DE3pLysE (available from Life Technology) was transformed by using each of the prepared plasmid samples. Transformed enzymes were produced by using each of the transformant samples and the obtained transformed enzymes were refined by using Ni-Agarose carriers.

### [Example 4. Identification of Compound I Degrading Enzyme]

Compound I was synthesized by a method that accords with the method described in Non-Patent Document 10. The NMR spectrum of the synthesized compound agreed with the data of Compound I published by USDA (US Forest Products Laboratory). A HPLC analysis of Compound I was executed by using Chiral Pack IE3 available from DAICEL under the condition of sequentially eluting erithro isomers of (1) αS, βR and (2) αR, βS and threo isomer of (3) αR, βR and (4) αS, βS in the above mentioned order in a manner as described below.

### Chiral HPLC Analysis Conditions (Conditions of Analyzing Optical Isomers of Compound I)

Column; Chiral Pack IE-3 (available from DAICEL), 4.6 mm I. d. × 250 mm L. eluent; (A) water, (B) acetonitrile, solution feeding rate; 20%V/V (B), column temperature; 40°C, flow rate: 1.0 ml/min, detection; Photodiode Array Detector UV200-500 nm (PDA model 2998, available from WATERS) and Optical Rotation Detector (CHIRALIZER, available from SYSTEM ENGINEERING)

The obtained Compound I showed a ratio of erithro isomers : threo isomers ≒ 3 : 1 under the above defined conditions. Note that the above RS expressions were determined by referring to the optical rotation data described in Non-Patent Document 11.

Compound I and NAD+ (added in the form of sodium salt) were added to a tris-HCl buffer solution (pH7.5) so as to make them show respective final concentrations of 5mM and 10mM. Subsequently, refined C01G1162, C01G1324, C10G0069, C10G0080, C10G0093 and C10G0094 that were obtained in Example 3 were added to the solution separately to produce so many different solutions and each of the solutions was incubated at room temperature for 16 hours to obtain a reaction solution. Thereafter, the change in Compound I in each of the reaction solutions was subjected to an HPLC analysis under the following conditions.

### Reverse Phase HPLC Conditions

Column; Xbridge OST C18 (available from WATERS), 4.6mm-I. d. × 100 mm-L, eluents; (A) [2mM ammonium acetate, 0.05%V/V formic acid], (B) methanol, solution feeding rate; 0-1 min 10%V/V(B), 1-8 min 10%V/V-90%V/V(B), column temperature; 40°C, flow rate; 1.2 ml/min, detection; Photodiode Array Detector UV200-500 nm (PDA model 2998, available from WATERS)

As a result of the HPLC analysis of each of the reaction solutions, Compound I was decreased only in the instances where C10G0069 or C10G0093 was caused to act to prove that a new compound had been produced in each of the above instances.

In order to identify the produced compound, each of the reaction solutions was refined by means of a solid phase extraction method (OASIS WAX; available from WATERS) and subjected to reverse phase UPLC-time-of-flight precision mass spectrometry (ACCUITY UPLC H-Class, XevoG2 QTOF, available form WATERS) under the following conditions.

### Reverse Phase HPLC Conditions (Reverse Phase UPLC-time-of Flight Precision mass spectrometry Conditions)

Column; ACQUITY UPLC BEH C18 Column, 130 angstroms, 1.7 µm, (available from WATERS), 2.1 mm I.d. × 100mm L. eluents; (A) [2mM ammonium acetate, 0.05%V/V formic acid], (B) 95%V/V acetonitrile, solution feeding rate; 0-5 minutes 5%V/V-95%V/V(B), 5-7 minutes 95%V/V(B), column temperature; 40°C. flow rate; 0.4 ml/min. Mass Spectrometry Conditions (Reverse Phase UPLC-time-of Flight Precision mass spectrometry Conditions)

Detection mass range 100-1,000 Da, data acquisition scanning interval 0.1 seconds, desolvation gas temperature 500°C, ion source ESI negative mode ion source temperature 150°C, cone voltage 30 V.

As a result of the reverse phase UPLC-time-of-flight precision mass spectrometry, it was found that the produced compound has a molecular weight of 318 and an estimated compositional formula of C₁₇H₁₈O₆. As a result of comparing the MS spectrum of Compound I and that of the produced compound, it was estimated that a dehydrogenation reaction had taken place at the Cα position of Compound I and the alcohol residue (hydroxyl group) had been changed to a carbonyl group to produce Compound II.

### [Example 5. Characteristics of Compound I Degrading Enzyme]

Compound II was synthesized according to the method described in Non-Patent Document 10. An HPLC analysis of Compound II was conducted under the condition of sequentially eluting (1) βR optical isomer and (2) βS optical isomer as described below, using a Chiral Pack IE3 available from DAICEL.

### Chiral HPLC Analysis Conditions (Conditions for Analyzing Optical Isomers of Compound II)

Column; Chiral Pack IE-3 (available from DAICEL), 4.6 mm I. d. × 250 mm L. eluents; (A) water, (B) acetonitrile, solution feeding rate; 30%V/V(B), column temperature; 40°C, flow rate; 1.0 ml/min, detection Photodiode Array Detector UV200-500 nm (PDA Model 2998, available from WATERS) and Chiralizer (available from SYSTEM ENGINEERING)

Under the above conditions, the synthesized Compound II showed a ratio of βR isomer : βS isomer ≒ 1 : 1. Note that the above RS expressions were determined by referring to the optical rotation data described in Non-Patent Document 11.

The synthesized Compound II was subjected to reverse phase UPLC-time-of-flight precision mass spectrometry under the conditions as described in Example 4. As a result, a mass chromatogram and a spectrum that agree quite well with those of the degraded product of Compound I obtained by causing C10G0069 and C10G0093 to act on Compound I were obtained. From this fact, it was found that C10G0069 and C10G0093 are proteins that show dehydrogenase activity of oxidizing the hydroxyl group at the Cα position of Compound I and turning the hydroxyl group into a carbonyl group.

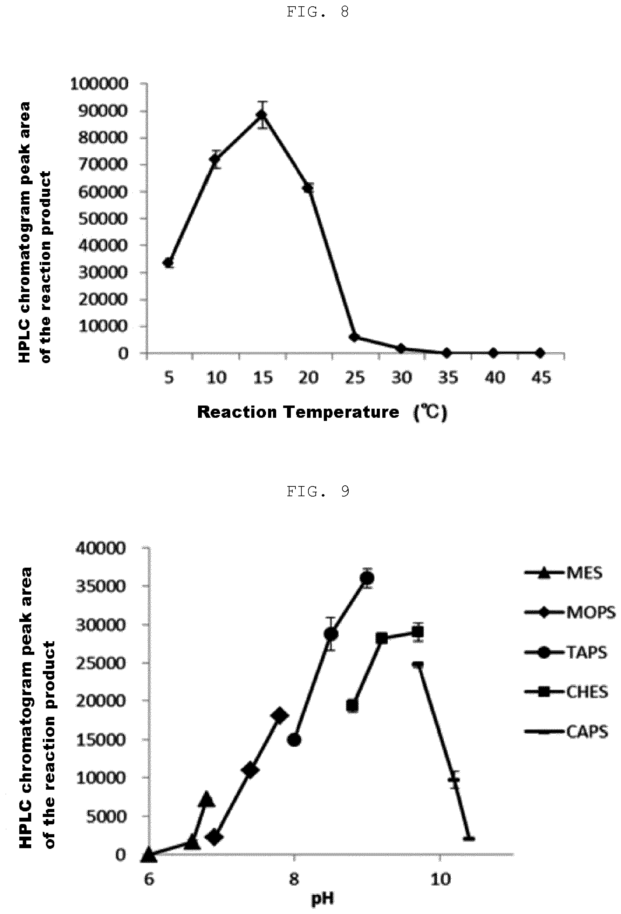

Reaction conditions under which C10G0069 acts on Compound I were looked into. C10G0069 oxidized the hydroxyl group at the Cα position when NAD+ coexisted. However, it did not show any activity when NAD+ did not exist. Additionally, it did not oxidize the hydroxyl group at the Cα position of Compound I in the presence of NADH but reduced the carbonyl residue of Compound II to produce a hydroxyl group. It did not show any activity in the presence of NADP+ and NADPH. Its molecular weight was determined to be 34kDa by means of SDS-PAGE. The optimum temperature of C10G0069 was estimated by causing a reaction solution (100 µl) containing C10G0069 by 10 mg/l, Compound I by 10 mM, NAD sodium by 20 mM and a (pH 9.2) N-Cyclohexyl-2-aminoethanesulfonic acid-NaOH (CHES) buffer solution (pH 9.5) to react at temperatures between 5 and 45°C for 30 minutes. As a result, it was found that the optimum temperature is somewhere between 10 and 15°C as shown in FIG. 8. Additionally, the optimum pH of C10G0069 was estimated by causing a reaction solution (100 µl) containing C10G0069 by 10 mg/l, Compound I by 10 mM, NAD sodium by 20 mM and a buffer solution (MES, MOPS, TAPS, CHES and CAPS) adjusted to pH between pH 6.0 and 10.4 by 50 mM to react at 15°C for 30 minutes. As a result, it was found the optimum pH is between 8.5 and 9.5 as shown in FIG. 9. Note that the optimum temperature and the optimum pH were based on the values obtained by analyzing the concentration of Compound II produced in the reaction solution after the reaction by means of reverse phase HPLC under the conditions described in Example 4.

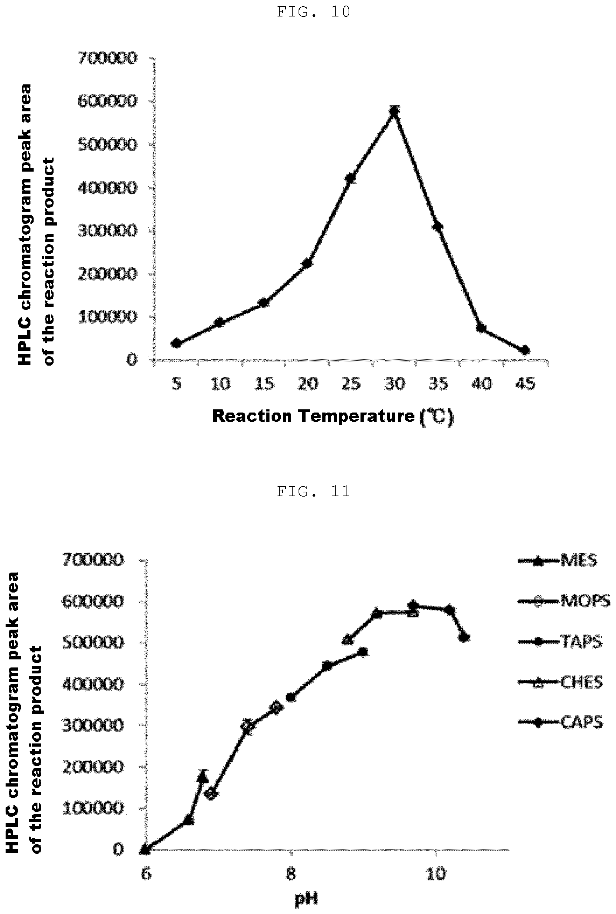

The reaction conditions under which C10G0093C acts on Compound I were looked into. C10G0093 oxidized the hydroxyl group at the Cα position of Compound I when NAD+ coexisted but did not show any activity when NAD+ did not exist. Additionally, C10G0093 did not oxidize the hydroxyl group at the Cα position of Compound I in the presence of NADH but reduced the carbonyl residue of Compound II to produce a hydroxyl group. It did not show any activity in the presence of NADP+ and NADPH. Its molecular weight was determined to be 34kDa by means of SDS-PAGE. The optimum temperature of C10G0093 was estimated by causing a reaction solution (100µl) containing C10G0093 by 5 mg/l, Compound I by 10mM, NAD sodium by 20mM and a (pH 9.2) CHES buffer solution (pH 9.5) by 50 mM to react at temperatures between 5 and 45°C for 30 minutes. As a result, it was found that the optimum temperature is somewhere between 25 and 30°C as shown in FIG. 10. Additionally, the optimum pH of C10G0093 was estimated by causing a reaction solution (100 µl) containing C10G0093 by 5 mg/l, Compound I by 10mM, NAD sodium by 20mM and a buffer solution (MES, MOPS, TAPS, CHES and CAPS) adjusted to pH between 6.0 and 10.4 by 50mM to react at 30°C for 30 minutes. As a result, it was found that the optimum pH is somewhere between 8.5 and 10.5 as shown in FIG. 11. Note that the optimum temperature and the optimum pH were based on the values obtained by analyzing the concentration of Compound II produced in the reaction solution after the reaction by means of reverse phase HPLC under the conditions described in Example 4.

Compound I has four optical isomers of αS, βR (SR); αR, βS (RS); αR, βR (RR); and αS, βS (SS). In order to find the optical isomers of Compound I on which C10C0069 and C10G0093 act as substrates out of the four isomers, the change in the composition of each of the optical isomers of Compound I was observed before and after the reaction and the composition of each of the optical isomers of Compound II, which is the reaction product, was subjected to an Chiral HPLC analysis under the following conditions.

### Chiral HPLC Analysis Conditions

Column; Chiral Pack IE 3 (available from DAICEL), 4.6 mm I. d. × 250 mm L. eluents; (A) water, (B) acetonitrile, solution feeding rate; 0-10 minutes 20%V/V (B), 10-15 minutes 20-30%V/V(B), 15-30 minutes 30%V/V (B), column temperature; 40°C, flow rate: 1.0 ml/min, detection; Photodiode Array Detector UV200-500nm (PDA model 2998, available from WATERS) and CHIRALIZER (available from

### SYSTEM ENGINEERING)

As a result of the Chiral HPLC analysis, it was found that C10G0069 specifically reacts to two optical isomers of RS and RR of Compound I and that C10G0093 specifically reacts to two optical isomers of SR and SS of C10G0093.

Additionally, most of the optical isomers of Compound I that are produced when Compound II is reduced in the presence of NADH was αR isomers in the case of using C10G0069 and αS isomers in the case of using C10G0093 (with an optical purity of >90% in terms of HPLC chromatogram peak area without sensitivity correction).

### [Example 6. Identification of Compound II Degrading Enzymes]

Compound I or Compound II and reduction type glutathione were added to a tris-hydrochloric acid buffer solution (pH 7.5) so as to make the respective final concentrations equal to 5 mM and 10 mM and subsequently each of refined proteins of C10G0075, C10G0076, C10G0077 and C10G078 obtained in Example 3 was added to the above admixture. Then, each of the obtained final mixtures was incubated at room temperature for 16 hours to obtain a reaction solution. Subsequently, the change, if any, that had taken place in the Compound I or Compound II in the related reaction solution was analyzed by means of reverse phase HPLC under the conditions described in Example 4.

As a result, it was found that, while Compound I does not change, Compound II is changed into two other compounds only when C10G0076 and C10G0077 are separately caused to act on it. A reverse phase HPLC analysis proved that one of the substances that were produced from Compound II is guaiacol from comparison of the retention time and the UV absorption spectrum of standard guaiacol and those of the produced substance. In order to estimate the chemical structure of the other compound produced from the reaction, the reaction solution was refined by means of a sold phase extraction method (OASIS WAX; available from WATERS) and subsequently the refined solution was subjected to reverse phase UPLC-time-of-flight precision mass spectrometry under the conditions described in Example 4.

As a result of the reverse phase UPLC-time-of-flight precision mass spectrometry (in negative ion mode), a signal of m/z500.2 was obtained from the substance produced from the reaction of C10G0076 or C10G0077 and Compound II. This signal substantially corresponds to the value obtained when a proton is desorbed from the molecular weight of 501.1 of the glutathione adduct of 3-hydroxy-1-(4-hydroxy-3-methoxyphenyl)-1-propanone (Compound III) that is reportedly produced when LigE and LigF of SYK-6 strain is caused to act on Compound I. In other words, it was determined that C10G0076 and C10G0077 have activity of cleaving the β-O-4 bond of Compound II just like LigE and LigF of SYK-6 strain.

### [Example 7, Degradation Characteristics of Compound II Degrading Enzymes]

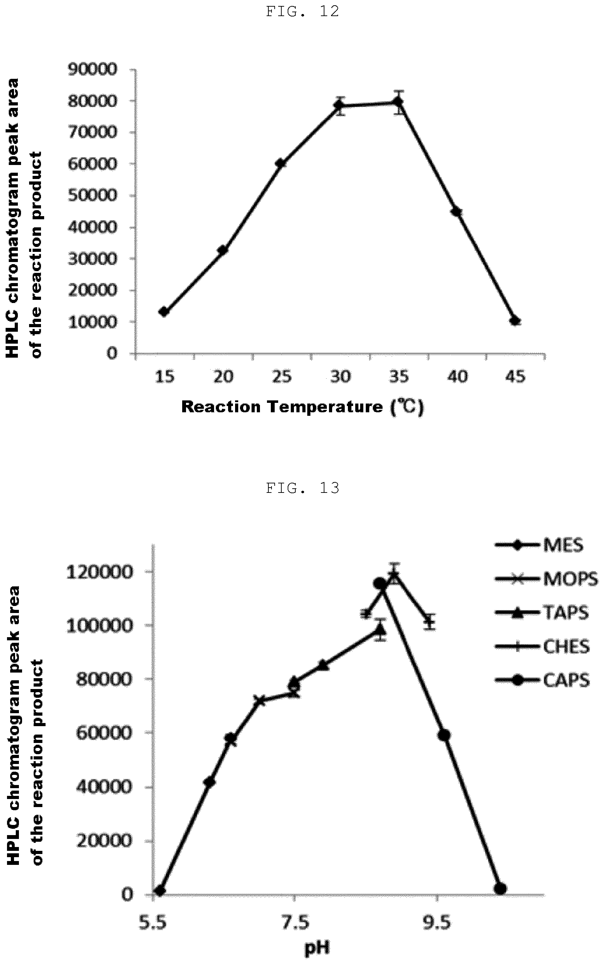

The reaction conditions when C10G0076 acts on Compound II were looked into. When reduction type glutathione is added so as to coexist in the reaction system, C10G0076 catalyzed cleavage of the β-O-4 bond due to the addition of glutathione. However, it did not show any activity of the type under consideration when reduction type glutathione did not exist in the reaction system. It was found by means of SDS-PAGE that the molecular weight is 31 kDa. The optimum temperature of C10G0076 was evaluated by causing a reaction solution (100 µl) containing C10G0076 by 5 mg/l, Compound II by 5 mM, reduction type glutathione by 10 mM and a (pH 8.9) CHES buffer solution (pH 9.5) by 100 mM at temperatures between 15 and 45°C for 30 minutes. As a result, it was found that the optimum temperature is between 30 and 35°C as shown in FIG. 12. Additionally, the optimum pH of C10G0076 was evaluated by causing a reaction solution (100 µl) containing C10G0076 by 5 mg/l, Compound II by 5 mM, reduction type glutathione by 10 mM and a buffer solution (MES, MOPS, TAPS, CHES and CAPS) adjusted to pH 5.6 to 10.4 by 50 mM at temperature of 30°C for 30 minutes. As a result, it was found that the optimum pH is somewhere between 8.5 and 9.5 as shown in FIG. 13. Note that the optimum temperature and the optimum pH are based on the values obtained by analyzing the concentration of the guaiacol produced in the reaction solution after the end of the reaction by means of reverse phase HPLC under the conditions (retention time around 5.5 minutes) described in Example 4.

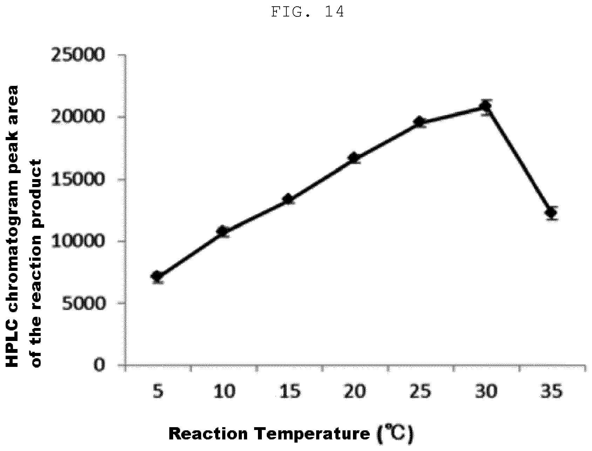

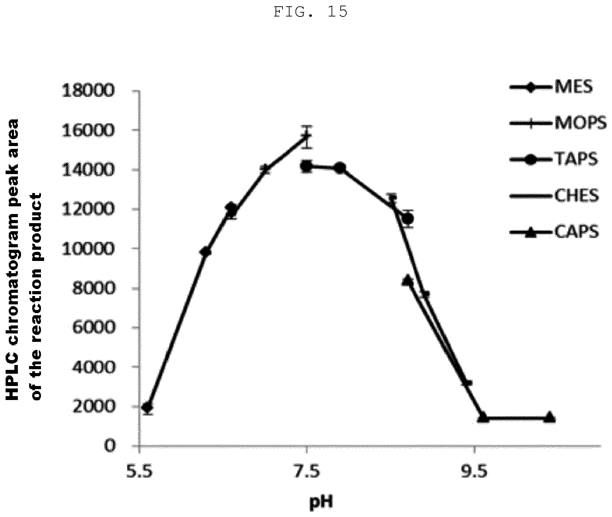

The reaction conditions under which C10G0077 acts on Compound II were looked into. When reduction type glutathione is added so as to coexist in the reaction system, C10G0077 catalyzed cleavage of the β-O-4 bond due to the addition of glutathione. However, it did not show any activity of the type under consideration when reduction type glutathione did not exist in the reaction system. It was found by means of SDS-PAGE that the molecular weight thereof is 31 kDa. The optimum temperature of C10G0077 was evaluated by causing a reaction solution (100 µl) containing C10G0077 by 5 mg/l, Compound II by 5 mM, reduction type glutathione by 10 mM and a (pH 8.9) MOPS buffer solution (pH 7.5) by 100 mM to react at temperatures between 5 and 35°C for 30 minutes. As a result, it was found that the optimum temperature is somewhere between 25 and 30°C as shown in FIG. 14. Additionally, the optimum pH of C10G0077 was evaluated by causing a reaction solution (100 µl) containing C10G0077 by 5 mg/l, Compound II by 5 mM, reduction type glutathione by 10 mM and a buffer solution (MES, MOPS, TAPS, CHES and CAPS) adjusted to pH 5.6 to 10.4 by 50 mM to react at temperature 20°C for 30 minutes. As a result, it was found that the optimum pH is somewhere between 7 and 8 as shown in FIG. 15. Note that the optimum temperature and the optimum pH are based on the values obtained by analyzing the concentration of the guaiacol produced in the reaction solution after the end of the reaction by means of reverse phase HPLC under the conditions described in Example 4.

Two different optical isomers of βR and βS exist in Compound II. In order to find the optical isomer or isomers on which C10G0076 and C10G0077 act as substrates out of them, the change in the composition of each of the optical isomers of Compound II that took place between before and after the reaction was observed and the composition of each of the optical isomers of Compound II, which is the reaction product, was subjected to an HPLC analysis by using a Chiral Column and a Chiral Pack IE-3. As a result, it was found that C10G0076 selectively acts on the βS isomer, while C10G0077 selectively acts on the βR isomer.

Additionally, that C10G0076 and C10G0077 are reactive relative to the non-phenolic lignin model dimer compound of Compound II was confirmed by using veratrylglycerol-β-guaiacylether(1-(3,4-dimethoxyphenyl)-2-(2-methoxyphenoxy)propane-1,3-diol (compound V) in the following manner. Note that ordinary lignins have non-phenolic constituent units to a large extent. Therefore, it will be safe to say that, enzymes that are active for degrading Compound V are probably also active for degrading natural lignins.

Compound V was synthesized by using, instead of the use of a conventional phase transfer catalyst, an improved method of employing N,N'-dimethylformamide (DMF) as solvent and adopting aldol reaction conditions of HCHO on a K₂CO₃-EtOH system by referring to the methods described in Non-Patent Documents 10 and 12. The NMR spectrum of the reaction product agreed with the data of Compound V that USDA publishes. As a result of calculations using the integrated value of β-proton on the ¹H-NMR spectrum, the ratio of the erithro isomers : the threo isomers was determined to be about 1.1 : 1.

A mixture solution of an 100mM (pH9.5) N-Cyclohexyl-2-aminoethanesulfonic acid-NaOH (CHES) buffer solution (pH9.5) containing C10G0076 by 0.1 µg, Compound V by 1 mM and reduction type glutathione by 2 mM and 10%V/V DMF was caused to react at 30°C for 15 minutes. After the reaction, the reaction solution was subjected to a reverse phase HPLC analysis to find that Compound V had been decreased and guaiacols had been generated. From this fact, it became clear that C10G0076 is reactive relative to non-phenolic lignin model dimer compounds. When C10G0076 was used, the efficiency of the reaction relative to Compound V, or a non-phenolic lignin model dimer compound, corresponded to 16.2% of the reaction efficiency relative to Compound II, or a phenolic lignin model dimer compound.

On the other hand, a mixture solution of an 100 mM 3-morpholinopropanesulfonic acid-NaOH (MOPS) buffer solution (pH 8.0) containing C10G0077 by 1.0 µg, Compound V by 1 mM and reduction type glutathione by 2 mM and 10%V/V DMF was caused to react at 20°C for 15 minutes. After the reaction, the reaction solution was subjected to a reverse phase HPLC analysis to find that, when C10G0077 was used, also Compound V had been decreased and guaiacol had been generated. As a result, it was made clear that C10G0077 is also reactive to non-phenolic lignin model dimer compounds. When C10G0077 was used, the efficiency of the reaction relative to Compound V, or a non-phenolic lignin model dimer compound, corresponded to 38.0% of the reaction efficiency relative to Compound II, or a phenolic lignin model dimer compound.

### [Example 8. Identification of Compound III Producing Enzymes]

A combination of refined proteins of C10G0075, C10G0076 and C10G0077 or refined proteins of C10G0076, C10G0077 and C10G0078, all of which were obtained in Example 3, were combined and caused to react, using Compound II as substrate. As a result, it was found that Compound III was produced from the reaction products of both of the combinations. Compound III showed a retention time that agree with the retention time of the metabolite (3-hydroxy-1-(4-hydroxy-3-methoxyphenyl)-1-propanone) that was obtained when Compound I was added to a culture solution of MBES04 strain. The reaction solution was refined by means of a solid phase extraction method (OASIS WAX; available from WATERS) and subjected to reverse phase UPLC-time-of-flight precision mass spectrometry under the conditions described in Example 4. The analysis results of Compound III agreed well with the analysis results of 3-hydroxy-1-(4-hydroxy-3-methoxyphenyl)-1-propanone, which is the principal product obtained when MBES04 strain was cultured on a culture medium containing Compound I.

That the compounds to which C10G0075 and C10G0078 react as substrate show optical isomer specificity was confirmed by combining the refined enzymes obtained in Example 3 in the following manner and causing transformation reactions of Compound II to take place. For the enzyme reactions, mixed solutions of 100 mM MOPS buffer solutions (pH 8.0) (each containing refined enzyme C10G0075 by 0.5 µg, refined enzyme C10G0076 by 0.5 µg, refined enzyme C10G0077 by 0.5 µg, refined enzyme C10G0078 by 0.5 µg per 100 µL of reaction solution), Compound II by 1 mM and reduction type glutathione by 10 mM, were caused to react at room temperature for 16 hours for the respective enzyme combinations of (1) through (4) shown below.
(1) C10G0076 (which specifically recognizes βS isomer) + C10G0075
(2) C10G0076 (which specifically recognizes (βS isomer) + C10G0078
(3) C10G0077 (which specifically recognizes βR isomer) + C10G0075
(4) C10G0077 (which specifically recognizes βR isomer) + C10G0078

After the reaction, the change in the substrate and the reaction product was detected by means of a Chiral HPLC analysis. As a result, the reaction proceeded and production of 3-hydroxy-1-(4-hydroxy-3-methoxyphenyl)-1-propanone was observed with each of the combinations of (1), (2) and (3). However, with the combination of (4), no production of 3-hydroxy-1-(4-hydroxy-3-methoxyphenyl)-1-propanone was observed. Thus, it was found that C10G0078 specifically recognizes only βS isomer, whereas C10G0075 can produce 3-hydroxy-1-(4-hydroxy-3-methoxyphenyl)-1-propanone from both of the optical isomers of βR and βS.

### [Example 9. Molecular weights of Compound III producing enzymes]

The molecular weight of C10G0078 was shown to be 33 kDa by means of SDS-PAGE. The molecular weight of C10G0078 was shown to be 27 kDa by means of SDS-PAGE.

### [Example 10. Lignin Degradation Using Compound I Degrading Enzymes, Compound II Degrading Enzymes and Compound III Degrading Enzymes (1)]

A compound having a phenyl propane structure was produced from oak sawdust extract solution. 50 g of dried and powdered oak sawdust was suspended in 300 mL of dioxane and left in an immersed condition at room temperature for 6 days. The solid content was removed from the suspension by means of filtration using a filter to obtain a filtrate. The filtrate was dried and solidified by means of an evaporator under reduced pressure to obtain dioxane extract of oak wood. The dioxane extract of oak wood was then dissolved in DMF so as to achieve a final concentration of 10%W/V. A reaction solution showing the following composition was prepared by using the obtained oak sawdust extract solution and caused to react at room temperature for 24 hours.

### Reaction solution (0.1 mL) composition (1):

Oak sawdust extract solution 1/20 capacity
(The concentration of the dried and solidified substance contained in the reaction solution was 5.0 mg/mL) CHES buffer solution pH 9.2 50 mM
Refined enzyme C10G0069 (protein concentration: 0.41 mg/mL) 1/20 capacity (5 µl)
Refined enzyme C10G0093 (protein concentration: 0.16 mg/mL) 1/20 capacity
Refined enzyme C10G0076 (protein concentration: 0.29 mg/mL) 1/20 capacity
Refined enzyme C10G0078 (protein concentration: 0.10 mg/mL) 1/20 capacity
Reduction type glutathione 5 mM
NAD sodium salt 5 mM

After the reaction, 50 µL of the reaction solution was refined by means of a solid phase extraction method (OASIS WAX; available from WATERS) and then dried and solidified in nitrogen gas. The residue obtained after the drying and solidifying process was dissolved in 0.5 mL of 20%V/V acetonitrile and subjected to reverse phase UPLC-time-of-flight precision mass spectrometry under the conditions described in Example 4.

As a result of the reverse phase UPLC-time-of-flight precision mass spectrometry, a signal of m/z195.1 was obtained at the retention time of 2.4 minutes due to an action of the added enzymes. The value of the retention time and that of the mass signal agreed very well with those of Compound III. The produced volume was calculated to be 1.1 µg/mL from the signal intensity of Compound III showing a known concentration. Additionally, a signal of m/z225.1 was obtained at the retention time of 2.5 minutes. The produced volume was calculated to be 0.8 µg/mL from the signal intensity of Compound III showing a known concentration. When the mass spectrum at the retention time of 2.4 minutes and the mass spectrum at the retention time of 2.5 minutes were compared, the compound (Compound IV) detected at 2.5 minutes was estimated to be a compound that is obtained by substituting a single hydrogen in Compound III with a methoxy group.

### [Example 11. Lignin Degradation Using Compound I Degrading Enzymes, Compound II Degrading Enzymes and Compound III Degrading Enzymes (2)]

A reaction solution having the following composition was prepared by using the oak sawdust extract solution prepared in Example 10 and caused to react at room temperature for 16 hours.

### Reaction solution (0.1 mL) composition (2):

Oak sawdust extract solution 1/20 capacity (The concentration of the dried and solidified substance contained in the reaction solution was 5.0 mg/mL)
TAPS buffer solution pH 8.5 100 mM
One of the combinations shown in Table 3 of the refined enzymes listed below.
Refined enzyme C10G0069 (protein concentration: 0.41 mg/mL) 1/20 capacity
Refined enzyme C10G0093 (protein concentration: 0.16 mg/mL) 1/20 capacity
Refined enzyme C10G0076 (protein concentration: 0.29 mg/mL) 1/20 capacity
Refined enzyme C10G0077 (protein concentration: 0.28 mg/mL) 1/20 capacity
Refined enzyme C10G0075 (protein concentration: 0.62 mg/mL) 1/20 capacity
Refined enzyme C10G0078 (protein concentration: 0.10 mg/mL) 1/20 capacity
Reduction type glutathione 5 mM
NAD sodium salt 5 mM

After the reaction, 50µL of the reaction solution was refined by means of a solid phase extraction method (OASIS WAX; available from WATERS) and then dried and solidified in nitrogen gas. The residue obtained after the drying and solidifying process was dissolved in 0.5 mL of 20%V/V acetonitrile and subjected to reverse phase UPLC-time-of-flight precision mass spectrometry under the conditions described in Example 4.

The amounts of Compound III produced under the effect of the added enzymes as observed by reverse phase UPLC-time-of-flight precision mass spectrometry are shown in Table 3. As seen from Table 3, Compound III was produced with the highest concentration when 6 enzymes were added.

**[Table 3]**

| | | **enzyme addition (+) / additive-free** (-) | | | | | | | |
|---|---|---|---|---|---|---|---|---|---|
| **Refined enzyme** | C 1 0 G 0 0 6 9 | - | + | + | + | + | + | + | + |
| **Refined enzyme** | C 1 0 G 0 0 9 3 | - | + | + | + | + | + | + | + |
| **Refined enzyme** | C 1 0 G 0 0 7 6 | - | - | - | + | + | + | + | + |
| **Refined enzyme** | C 1 0 G 0 0 7 7 | - | + | + | - | - | + | + | + |
| **Refined enzyme** | C 1 0 G 0 0 7 5 | - | - | + | - | + | - | + | + |
| **Refined enzyme** | C 1 0 G 0 0 7 8 | - | + | - | + | - | + | - | + |
| **Concentration of Compound III after the reaction (µg/mL)** | | 0.0 | 0.0 | 7.1 | 3.4 | 3.2 | 7.3 | 9.9 | 11.8 |

### [Example 12. Degradation of Lignins with Use of Compound I Degrading Enzymes, Compound II Degrading Enzymes and Compound III Degrading Enzymes (3)]

50 g of dried and powdered rice straws was suspended in a mixture solution of 300 mL of dioxane and 15 mL of water and left in an immersed condition at room temperature for 6 days. After the immersion, impurities were filtered out from the suspension by means of filter paper to obtain a filtrate. The filtrate was dried and solidified by means of an evaporator under reduced pressure and subsequently dried out by means of a desiccator to obtain 2.65 g of solid. 50 mL of ethyl acetate and 70 mL of pure water were added to the solid in order to dissolve the solid in the mixture solution. Additionally, the solid was further dissolved in DMF so as to achieve a final concentration of 10%W/V. A reaction solution showing the following composition was prepared, using the obtained rice straw extract DMF solution, and caused to react at room temperature for 24 hours. Reaction solution (0.1 mL) composition (3):
Rice straw extract DMF solution 1/20 capacity
(The concentration of the dried and solidified substance contained in the reaction solution was 5.0 mg/mL)
CHES buffer solution pH 9.2 50 mM
Refined enzyme C10G0069 (protein concentration: 0.41 mg/mL) 1/20 capacity
Refined enzyme C10G0093 (protein concentration: 0.16 mg/mL) 1/20 capacity
Refined enzyme C10G0075 (protein concentration: 0.62 mg/mL) 1/20 capacity
Refined enzyme C10G0076 (protein concentration: 0.29 mg/mL) 1/20 capacity
Refined enzyme C10G0077 (protein concentration: 0.28 mg/mL) 1/20 capacity
Reduction type glutathione 5 mM
NAD sodium salt 5 mM

After the reaction, 50 µL of the reaction solution was refined by means of a solid phase extraction method (OASIS WAX; available from WATERS) and then dried and solidified in nitrogen gas. The residue obtained after the drying and solidifying process was dissolved in 0.5 mL of 20%V/V acetonitrile and subjected to reverse phase UPLC-time-of-flight precision mass spectrometry under the conditions described in Example 4.

As a result of the reverse phase UPLC-time-of-flight precision mass spectrometry, it was confirmed that Compound III and Compound IV had been produced under the effect of the added enzymes. The amounts of the produced compounds were respectively 8.4 µg/mL and 0.7 µg/mL.

### [Example 13. Degradation of Lignins with Use of Compound I Degrading Enzymes, Compound II Degrading Enzymes and Compound III Degrading Enzymes (3)]

A compound having a phenyl propane structure obtained from a waste Shiitake mushroom bed was prepared by way of the following steps. 10 g of the powdered waste Shiitake mushroom bed was suspended in 100 mL of ion exchange water and left in an immersed condition at room temperature for 3 hours. The solid ingredients were filtered out from the obtained suspension by means of filter paper to obtain a filtrate (water-washed solution 1). The residue was suspended in 100 mL of isopropanol aqueous solution and left in an immersed condition at room temperature for 3 hours. After the immersion, the solid ingredients were filtered out by means of filter paper to obtain a filtrate (post-water-washing isopropanol solution 1). On the other hand, 10 g of the dried and powdered waste Shiitake mushroom bed was suspended in 100 mL of 90%V/V isopropanol aqueous solution and left in an immersed condition at room temperature for 3 hours. The solid ingredients were filtered out by means of filter paper to obtain a filtrate (90% isopropanol solution 1).

25 mL was taken out from each of the water-washed solution 1, the post-water-washing isopropanol solution 1 and the 90% isopropanol solution 1 and each of the sample solutions was dried and solidified under reduced pressure, while being heated in an evaporator at 45°C. As a result, solids were obtained respectively by 469 mg, by 55 mg and by 247 mg from the water-washed solution 1, the post-water-washing isopropanol solution 1 and the 90% isopropanol solution. The solid obtained from the water-washed solution 1 was dissolved in 2 mL of water (234.4 mg/mL), while each of the solids obtained from the post-water-washing isopropanol solution 1 (27.4 ml/mL) and the 90% isopropanol solution 1 (123.5 mg/mL) was dissolved in 2 mL of DMF. Reaction solutions having the compositions as described below were prepared by respectively using these three solution-extracts and caused to react at room temperature for 24 hours.

### Reaction solution composition (4):

Waste Shiitake mushroom bed extract solution (one of the above-listed three extracts) 1/20 capacity
(The concentration of the dried and solidified substance contained in the reaction solution was 11.7 mg/mL when the water-washed solution 1 was used, 1.37 mg/mL when the post-water-washing isopropanol solution 1 was used and 6.18 mg/mL when the 90% isopropanol solution 1 was used.) CHES buffer solution pH 9.2 50 mM
Refined enzyme C10G0069 (protein concentration: 0.41 mg/mL) 1/20 capacity
Refined enzyme C10G0093 (protein concentration: 0.16 mg/mL) 1/20 capacity
Refined enzyme C10G0075 (protein concentration: 0.62 mg/mL) 1/20 capacity
Refined enzyme C10G0076 (protein concentration: 0.29 mg/mL) 1/20 capacity
Refined enzyme C10G0077 (protein concentration: 0.28 mg/mL) 1/20 capacity
Reduction type glutathione 5 mM
NAD sodium salt 5 mM

After the reactions, 50 µL of each of the reaction solutions was refined by means of a solid phase extraction method (OASIS WAX; available from WATERS) and then dried and solidified in nitrogen gas. The residue obtained after the drying and solidifying process was dissolved in 0.5 mL of 20%V/V acetonitrile and subjected to reverse phase UPLC-time-of-flight precision mass spectrometry under the conditions described in Example 4.

As a result of the reverse phase UPLC-time-of-flight precision mass spectrometry, it was confirmed that Compound III had been produced under the effect of the added enzymes for all the solutions. The amount of the produced compound was 1.1 µg/mL when the water-washed solution 1 was used, 0.68 µg/mL when the post-water-washing isopropanol solution 1 was used and 1.37 µg/mL when the 90% isopropanol solution 1 was used. The produced amount of Compound IV was 0.6 µg/mL when the water-washed solution 1 was used, 0.3 µg/mL when the post-water-washing isopropanol solution 1 was used and 0.7 µg/mL when the 90% isopropanol solution 1 was used.

### [Reference Documents]

The above cited Non-Patent Documents 4 through 12 are listed below.:
Non-Patent Document 4: Besemer J et al.;Nucleic Acids Research, 2001, vol.29, p.2607
Non-Patent Document 5: Masai, E., S. Kubota. Y. Katayama, S. Kawai, M. Yamasaki, and N. Morohoshi. 1993. Biosci.
Biotechnol. Biochem. 57:1655-1659
Non-Patent Document 6: Sato Y, Moriuchi H, Hishiyama S, Otsuka Y, Oshima K, Kasai D, Nakamura M, Ohara S, Katayama Y, Fukuda M, Masai E. Appl Environ Microbiol. 2009; 75 (16):5195-201.
Non-Patent Document 7: Masai, E., Y. Katayama, S. Kubota, S. Kawai, M. Yamasaki, and N. Morohoshi. 1993. FEBS Lett. 323: 135-140.
Non-Patent Document 8: Masai, E., Y. Katayama, S. Kawai, S. Nishikawa, M. Yamasaki, and N. Morohoshi. 1991. J. Bacteriol. 173: 7950-7955.
Non-Patent Document 9: Masai E, Ichimura A, Sato Y, Miyauchi K, Katayama Y, Fukuda M. J Bacteriol. 2003 Mar; 185 (6):1768-75.
Non-Patent Document 10: Hosoya et al., Mokuzai Gakkaishi 26 (2) 118-21, 1980
Non-Patent Document 11: Hishiya et al.:Tetrahedron Letters 53, 842-845, 2012
Non-Patent Document 12: K.Itoh :Mokuzai Gakkaishi vol.38, No.6, 579-584 (1992)

The sequences as described in the related sequence listings are as follow.
[Sequence Number 1] C10G0069
[Sequence Number 2] C10G0093
[Sequence Number 3] C10G0076
[Sequence Number 4] C10G0077
[Sequence Number 5] C10G0078
[Sequence Number 6] C10G0075
[Sequence Number 7] Gene c10g0069
[Sequence Number 8] Gene c10g0093
[Sequence Number 9] Gene c10g0076
[Sequence Number 10] Gene c10g0077
[Sequence Number 11] Gene c10g0078
[Sequence Number 12] Gene c10g0075
[Sequence Number 13] Primer A
   TCTCGAGCTCGGATCC
[Sequence Number 14] Primer B
   CTGGTACCATGGAATTCG
[Sequence Number 15] Primer C
   GGATCCGAGCTCGAGATGATCAAGGGTATCGAAGG
[Sequence Number 16] Primer D
   CGAATTCCATGGTACCAGTCAGAACTCCTGTGCGG
[Sequence Number 17] Primer E
   GGATCCGAGCTCGAGATGACGAACTGGCTTATCAC
[Sequence Number 18] Primer F
   CGAATTCCATGGTACCAGTCAGACCTCGGCGAAG
[Sequence Number 19] Primer G
   GGATCCGAGCTCGAGATGACACAGGTAAAGGGACG
[Sequence Number 20] Primer H
   CGAATTCCATGGTACCAGTCATGCCGTCTTTTCCTC
[Sequence Number 21] Primer I
   GGATCCGAGCTCGAGATGGGAGAGACGACAAAAC
[Sequence Number 22] Primer J
   CGAATTCCATGGTACCAGTCAGGTGAGGTCGGC
[Sequence Number 23] Primer K
   GGATCCGAGCTCGAGATGCAGGATCTACCGGG
[Sequence Number 24] Primer L
   CGAATTCCATGGTACCGCAAGCTGTGTCATGC
[Sequence Number 25] Primer M
   GGATCCGAGCTCGAGATGACGGGCGGGG
[Sequence Number 26] Primer N
   CGAATTCCATGGTACCAGTCAGAGCGCGTTGGC
[Sequence Number 27] Primer O
   GGATCCGAGCTCGAGATGCTGGAACTGTGGACTTC
[Sequence Number 28] Primer P
   CGAATTCCATGGTACCAGGTAGGTGTGCTCATCGTTCA
[Sequence Number 29] Primer Q
   GGATCCGAGCTCGAGATGTTGACGCTGTACAGCTTTG
[Sequence Number 30] Primer R
   CGAATTCCATGGTACCAGCTCCTCAGGCCTGTGC
[Sequence Number 31] Primer S
   GGATCCGAGCTCGAGATGGCCAAGGACAACC
[Sequence Number 32] Primer T
   CGAATTCCATGGTACCAGTCAGCTCGCCGTAGC
[Sequence Number 33] Primer U
   GGATCCGAGCTCGAGATGGCATGGGACGATG
[Sequence Number 34] Primer V
   CGAATTCCATGGTACCAGGATGACGGTGTGCTTCAC

### INDUSTRIAL APPLICABILITY

3-hydroxy-1-(4-hydroxy-3-methoxyphenyl)-1-propanone can be obtained from biomass containing natural lignins and lignin-related substances by means of the production method and the enzymes according to the present invention. 3-hydroxy-1-(4-hydroxy-3-methoxyphenyl)-1-propanone can be transformed into various industrially useful compounds. For example, it can be utilized as starting material for producing resins, adhesive agents, resist materials, drugs and so on. It can also be utilized as starting material for producing 1-(4-hydroxy-3-methoxyphenyl)-1, 3-propanediol and other chemical compounds.

## Claims

1. A method for producing a phenyl propane-based compound comprising a step of producing a phenyl propane-based compound by causing enzymes derived from microorganisms of the genus Novosphingobium to act on biomass containing lignins in the presence of NAD and reduction type glutathione, wherein
the microorganisms of the genus Novosphingobium are Novosphingobium species MBES04 (deposition number: NITE AP-01797), and
the enzymes are a combination of enzymes described in (1) shown below and enzymes described in (2) shown below as well as enzymes described in (3) shown below and/or enzymes described in (4) shown below:
(1) Short-chain dehydrogenase/reductase that belongs to the Rossmann-fold NAD(P)(+) binding protein super family, has Multi-domain of PRK06194 and oxidizes the Cα carbon of guaiacylglycerol-β-guaiacyl ether;
(2) Glutathione S-transferase that has Multi-domain of PRK 15113 and maiA at the side of Gst and the N-end;
(3) Glutathione S-transferase that has Multi-domain of PRK 10387 and maiA at the side of Gst and the N-end;
(4) Glutathione S-transferase that has Multi-domain of Gst, PRK11752, PRK15113 and maiA; and
the enzyme of (1) is an enzyme having an amino acid sequence with at least (≥) 90% identity to an amino acid sequence as defined in the sequence listing with sequence number 1 or sequence number 2; the enzyme of (2) is either C10G0076 having an amino acid sequence as defined in the sequence listing with sequence number 3 or C10G0077 having an amino acid sequence as defined in the sequence listing with sequence number 4; the enzyme of (3) is C10G0078 having an amino acid sequence as defined in the sequence listing with sequence number 5; and the enzyme of (4) is C10G0075 having an amino acid sequence as defined in the sequence listing with sequence number 6; and
the phenyl propane-based compound is expressed by general formula (A) 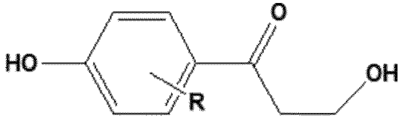 where R represents 1, 2 or morealkyl groups or alkoxy groups having 1 to 5 carbon atoms or hydrogen atoms.

2. The method according to claim 1, wherein the phenyl propane-based compound is one selected from a group of 3-hydroxy-1-(4-hydroxy-3-methoxyphenyl)-1-propanone, 3-hydroxy-1-(4-hydroxy-3, 5-dimethoxyphenyl)-1-propanone and 3-hydroxy-1-(4-hydroxyphenyl)-1-propanone.

3. The method according to claim 1, wherein the phenyl propane-based compound is 3-hydroxy-1-(4-hydroxy-3-methoxyphenyl)-1-propanone.

4. The method according to any one of claims 1 to 3, wherein the enzyme of (1) is either C10G0069 having an amino acid sequence as defined in the sequence listing with sequence number 1 or C10G0093 having an amino acid sequence as defined in the sequence listing with sequence number 2.

5. A short-chain dehydrogenase/reductase that is derived from microorganisms of the genus Novosphingobium and belongs to the Rossmann-fold NAD(P)(+) binding protein super family, while it has Multi-domain of PRK06194 and oxidizes the Cα carbon of guaiacylglycerol-β-guaiacyl ether, wherein
the microorganisms of the genus Novosphingobium are Novosphingobium species MBES04 (deposition number: NITE AP-01797), and
the short-chain dehydrogenase/reductase is either C10G0069 having an amino acid sequence as defined in the sequence listing with sequence number 1 or C10G0093 having an amino acid sequence as defined in the sequence listing with sequence number 2.

## Patentansprüche

1. Ein Verfahren zur Herstellung einer Phenylpropan-basierten Verbindung, umfassend einen Schritt der Herstellung einer Phenylpropan-basierten Verbindung, indem bewirkt wird, dass von Mikroorganismen der Gattung Novosphingobium abgeleitete Enzyme auf Lignine enthaltende Biomasse in Gegenwart von NAD und Reduktionstyp-Glutathion einwirken, wobei
die Mikroorganismen der Gattung Novosphingobium Novosphingobium Spezies MBES04 (Hinterlegungsnummer: NITE AP-01797) sind, und
die Enzyme eine Kombination aus den Enzymen sind, die in (1) weiter unten gezeigt sind, und den Enzymen, die in (2) weiter unten gezeigt sind, sowie den Enzymen, die in (3) weiter unten gezeigt sind und/oder den Enzymen, die in (4) weiter unten gezeigt sind:
(1) (1) Kurzkettige Dehydrogenase/Reduktase, die zu der Rossmann-Faltung NAD(P)(+) Bindeprotein Superfamilie gehört, Multi-Domäne von PRK06194 aufweist und den Cα-Kohlenstoff von Guaiacylglycerin-β-Guaiacylether oxidiert;
(2) Glutathion-S-Transferase, die Multi-Domäne von PRK 15113 und maiA an der Seite von Gst und dem N-Ende aufweist;
(3) Glutathion-S-Transferase, die Multi-Domäne von PRK 10387 und maiA an der Seite von Gst und dem N-Ende aufweist;
(4) Glutathion-S-Transferase, die Multi-Domäne von Gst, PRK11752, PRK15113 und maiA aufweist; und
das Enzym von (1), ein Enzym ist, das eine Aminosäuresequenz mit mindestens (≥) 90% Identität zu einer Aminosäuresequenz aufweist, wie sie in dem Sequenzprotokoll mit der Sequenznummer 1 oder Sequenznummer 2 definiert ist; das Enzym von (2) entweder C10G0076 ist, das eine Aminosäuresequenz aufweist, wie sie in dem Sequenzprotokoll mit der Sequenznummer 3 definiert ist oder C10G0077, das eine Aminosäuresequenz aufweist, wie sie in dem Sequenzprotokoll mit der Sequenznummer 4 definiert ist; das Enzym von (3) C10G0078 ist, das eine Aminosäuresequenz aufweist, wie sie in dem Sequenzprotokoll mit der Sequenznummer 5 definiert ist; und das Enzym von (4) C10G0075 ist, das eine Aminosäuresequenz aufweist, wie sie in dem Sequenzprotokoll mit der Sequenznummer 6 definiert ist; und
die Phenylpropan-basierte Verbindung durch die allgemeine Formel (A) ausgedrückt ist 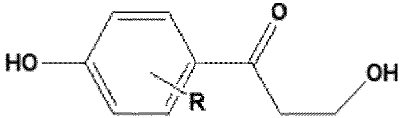
worin R 1, 2 oder mehr Alkyl-Gruppen oder Alkoxy-Gruppen, aufweisend 1 bis 5 Kohlenstoff-Atomen oder Wasserstoff-Atomen, darstellt.

2. Das Verfahren gemäß Anspruch 1, wobei die Phenylpropan-basierte Verbindung aus einer Gruppe von 3-Hydroxy-1-(4-hydroxy-3-methoxyphenyl)-1-propanon, 3-Hydroxy-1-(4-hydroxy-3,5-dimethoxyphenyl)-1-propanon und 3-Hydroxy-1-(4-hydroxyphenyl)-1-propanon ausgewählt ist.

3. Das Verfahren gemäß Anspruch 1, wobei die Phenylpropan-basierte Verbindung 3-Hydroxy-1-(4-hydroxy-3-methoxyphenyl)-1-propanon ist.

4. Das Verfahren gemäß einem der Ansprüche 1 bis 3, wobei das Enzym von (1) entweder C10G0069 ist, das eine Aminosäuresequenz aufweist, wie sie in dem Sequenzprotokoll mit der Sequenznummer 1 definiert ist, oder C10G0093, das eine Aminosäuresequenz aufweist, wie sie in dem Sequenzprotokoll mit der Sequenznummer 2 definiert ist.

5. Eine kurzkettige Dehydrogenase/Reduktase, die von Mikroorganismen der Gattung Novosphingobium abgeleitet ist und zu der Rossmann-Faltung NAD(P)(+) Bindeprotein Superfamilie gehört, während es Multi-Domäne von PRK06194 aufweist und den Cα-Kohlenstoff von Guaiacylglycerol-β-guaiacylether oxidiert, wobei
die Mikroorganismen der Gattung Novosphingobium Novosphingobium Spezies MBES04 (Hinterlegungsnummer: NITE AP-01797) sind, und
die kurzkettige Dehydrogenase/Reduktase entweder C10G0069 ist, die eine Aminosäuresequenz aufweist, wie sie in dem Sequenzprotokoll mit der Sequenznummer 1 definiert ist, oder C10G0093, die eine Aminosäuresequenz aufweist, wie sie in dem Sequenzprotokoll mit der Sequenznummer 2 definiert ist.

## Revendications

1. Procédé de production d'un composé à base de phénylpropane comprenant une étape consistant à produire un composé à base de phénylpropane en amenant des enzymes dérivées de microorganismes du genre Novosphingobium à agir sur de la biomasse contenant des lignines en présence de NAD et de glutathion de type à réduction, dans lequel
les microorganismes du genre Novosphingobium sont Novosphingobium espèce MBES04 (numéro de dépôt : NITE AP-01797), et
les enzymes sont une combinaison d'enzymes décrites en (1) illustré ci-dessous et d'enzymes décrites en (2) illustré ci-dessous ainsi que d'enzymes décrites en (3) illustré ci-dessous et/ou d'enzymes décrites en (4) illustré ci-dessous :
(1) déshydrogénase/réductase à chaîne courte qui appartient à la superfamille de protéines de liaison à NAD(P)(+) à pli Rossmann, a le multi-domaine de PRK06194 et oxyde le carbone Cα du guaiacylglycérol-β-guaiacyle éther ;
(2) glutathion S-transférase qui a le multi-domaine de PRK 15113 et maiA sur le côté de Gst et l'extrémité N ;
(3) glutathion S-transférase qui a le multi-domaine de PRK 10387 et maiA sur le côté de Gst et l'extrémité N ;
(4) glutathion S-transférase qui a le multi-domaine de Gst, PRK11752, PRK15113 et maiA ; et
l'enzyme de (1) est une enzyme ayant une séquence d'acides aminés avec au moins (≥) 90 % d'identité à une séquence d'acides aminés telle que définie dans la liste de séquences avec le numéro de séquence 1 ou numéro de séquence 2, l'enzyme de (2) est soit C10G0076 ayant une séquence d'acides aminés telle que définie dans la liste de séquences avec le numéro de séquence 3, soit C10G0077 ayant une séquence d'acides aminés telle que définie dans la liste de séquences avec le numéro de séquence 4 ; l'enzyme de (3) est C10G0078 ayant une séquence d'acides aminés telle que définie dans la liste de séquences avec le numéro de séquence 5 ; et l'enzyme de (4) est C10G0075 ayant une séquence d'acides aminés telle que définie dans la liste de séquences avec le numéro de séquence 6 ; et
le composé à base de phénylpropane est exprimé par la formule générale (A) 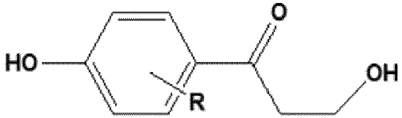
où R représente 1, 2 groupes alkyle ou groupes alcoxy ou plus ayant 1 à 5 atomes de carbone ou atomes d'hydrogène.

2. Procédé selon la revendication 1, dans lequel le composé à base de phénylpropane est un composé sélectionné parmi un groupe du 3-hydroxy-1-(4-hydroxy-3-méthoxyphényl)-1-propanone, 3-hydroxy-1-(4-hydroxy-3,5-diméthoxyphényl)-1-propanone et 3-hydroxy-1-(4-hydroxyphényl)-1-propanone.

3. Procédé selon la revendication 1, dans lequel le composé à base de phénylpropane est le 3-hydroxy-1-(4-hydroxy-3-méthoxyphényl)-1-propanone.

4. Procédé selon l'une quelconque des revendications 1 à 3, dans lequel l'enzyme de (1) est soit C10G0069 ayant une séquence d'acides aminés telle que définie dans la liste de séquences avec le numéro de séquence 1, soit C10G0093 ayant une séquence d'acides aminés telle que définie dans la liste de séquences avec le numéro de séquence 2.

5. Déshydrogénase/réductase à chaîne courte qui est dérivée de microorganismes du genre Novosphingobium et appartient à la superfamille de protéines de liaison à NAD(P)(+) à pli Rossmann, tandis qu'elle a le multi-domaine de PRK06194 et oxyde le carbone Cα du guaiacylglycérol-β-guaiacyle éther, dans laquelle
les microorganismes du genre Novosphingobium sont Novosphingobium espèce MBES04 (numéro de dépôt : NITE AP-01797), et
la déshydrogénase/réductase à chaîne courte est soit C10G0069 ayant une séquence d'acides aminés telle que définie dans la liste de séquences avec le numéro de séquence 1, soit C10G0093 ayant une séquence d'acides aminés telle que définie dans la liste de séquences avec le numéro de séquence 2.


### SEQUENCE LISTING

<110> Japan Agency for Marine-Earth Science and Technology (JAMSTEC)
<120> A method to produce phenylpropane compouds using enzymes
<130> 15DF0306PCT
<160> 34
<170> PatentIn version 3.5
<210> 1
   <211> 303
   <212> PRT
   <213> Artificial sequence
<220>
   <223> Enzyme
<400> 1
   Met Thr Gln Val Lys Gly Arg Thr Ala Phe Ile Thr Gly Gly Gly Ser
   1               5                   10                  15
   Gly Val Ala Leu Gly Gln Ala Lys Val Phe Ala Arg Ala Gly Cys Lys
               20                  25                  30
   Val Ala Ile Ala Asp Ile Arg Gln Asp His Leu Asp Glu Ala Met Ala
           35                  40                  45
   Trp Phe Glu Ala Glu Asn Ala Lys Gly Ala Asn Tyr Glu Val Met Ala
       50                  55                  60
   Val Lys Leu Asp Ile Thr Asp Arg Glu Ala Tyr Ala Lys Val Ala Asp
   65                  70                  75                  80
   Glu Val Glu Ala Lys Leu Gly Pro Val Glu Leu Leu Phe Asn Thr Ala
                   85                  90                  95
   Gly Val Ser His Phe Gly Ala Ile Gln Asp Ala Thr Tyr Asp Asp Trp
               100                 105                 110
   Asp Trp Gln Ile Asp Val Asn Leu Arg Gly Val Ile Asn Gly Val Arg
           115                 120                 125
   Thr Phe Val Pro Arg Met Ile Glu Arg Gly Asn Gly Gly His Val Val
       130                 135                 140
   Asn Thr Ala Ser Met Ser Ala Phe Val Ala Leu Lys Gly Thr Gly Ile
   145                 150                 155                 160
   Tyr Cys Thr Thr Lys Met Ala Val Arg Gly Leu Thr Glu Thr Leu Ala
                   165                 170                 175
   Leu Asp Leu Glu Glu His Gly Ile Gly Val Ser Leu Leu Cys Pro Gly
               180                 185                 190
   Ala Val Asn Thr Asn Ile His Glu Ala Leu Leu Thr Arg Pro Lys His
           195                 200                 205
   Leu Ala Asp Thr Gly Tyr Tyr Gln Ala Gly Pro Glu Met Phe Ala His
       210                 215                 220
   Leu Lys Asn Val Ile Glu Cys Gly Met Glu Pro Glu Thr Leu Ala Asn
   225                 230                 235                 240
   His Val Leu Lys Ala Val Glu Glu Asn Gln Leu Tyr Val Leu Ala Tyr
                   245                 250                 255
   Pro Glu Phe Arg Lys Pro Leu Glu Asp Ile His Ala Arg Val Met Ala
               260                 265                 270
   Ala Leu Ala Asn Pro Glu Asp Asp Pro Asp Tyr Asp Arg Arg Val Ala
           275                 280                 285
   His Gly Val Pro Gly Gly Glu Ala Lys Glu Glu Glu Lys Thr Ala
       290                 295                 300
<210> 2
   <211> 311
   <212> PRT
   <213> Artificial sequence
<220>
   <223> Enzyme
<400> 2
   Met Gln Asp Leu Pro Gly Lys Thr Ala Phe Val Thr Gly Gly Ala Ser
   1               5                   10                  15
   Gly Ile Gly Leu Gly Ile Ala Lys Ala Leu Leu Gly Ala Gly Met Asn
               20                  25                  30
   Val Ala Ile Ala Asp Ile Arg Gln Asp His Leu Asp Asp Ala Ala Ala
           35                  40                  45
   Glu Leu Asp Gly Gly Asp Lys Val Leu Ala Leu Gln Leu Asp Val Thr
       50                  55                  60
   Asp Arg Ala Ala Phe Ala Ala Ala Ala Asp Ala Thr Glu Ala Lys Phe
   65                  70                  75                  80
   Gly Lys Ile His Ile Leu Cys Asn Asn Ala Gly Val Ala Val Val Gly
                   85                  90                  95
   Pro Thr Asp Met Ala Thr Phe Ala Asp Trp Asp Trp Val Met Gly Val
               100                 105                 110
   Asn Val Gly Gly Thr Ile Asn Gly Ile Val Thr Met Leu Pro Arg Met
           115                 120                 125
   Leu Lys His Gly Glu Gly Gly His Ile Val Asn Thr Ala Ser Met Ser
       130                 135                 140
   Ala Leu Val Pro Ala Pro Gly Thr Thr Ile Tyr Ser Ser Gly Lys Ala
   145                 150                 155                 160
   Ala Val Thr Ser Met Met Glu Cys Met Arg Pro Glu Leu Glu Ser Arg
                   165                 170                 175
   Gly Ile Val Cys Ser Ala Phe Cys Pro Gly Ala Val Gln Ser Asn Ile
               180                 185                 190
   Ala Glu Ala Gly Arg Thr Arg Pro Asp Ala Leu Ala Glu Thr Gly Tyr
           195                 200                 205
   Ala Glu Ala Asp Lys Gly Arg Gln Ala Gly Gly Ser Phe Phe His Leu
       210                 215                 220
   Tyr Gln Thr Lys Glu Glu Val Gly Glu Arg Val Leu Thr Gly Ile Leu
   225                 230                 235                 240
   Asn Asp Glu Leu Tyr Ile Leu Thr His Ala Glu Phe Leu Ile Gly Val
                   245                 250                 255
   Gln Glu Arg Gly Glu Ala Thr Thr Ala Ala Val Gln Val Gln Leu Pro
               260                 265                 270
   Glu Asn Glu Glu Tyr Lys Asn Thr Phe Gly Val Leu Phe Arg Asn Ser
           275                 280                 285
   Ala Ile Thr Gln Glu Ile Asp Arg Gln Lys Ala Leu Arg Ala Ala Gln
       290                 295                 300
   Met Ser Glu Ala Gly Val Ala
   305                 310
<210> 3
   <211> 256
   <212> PRT
   <213> Artificial sequence
<220>
   <223> Enzyme
<400> 3
   Met Leu Thr Leu Tyr Ser Phe Gly Pro Gly Ala Asn Ser Leu Lys Pro
   1               5                   10                  15
   Leu Leu Ala Leu Tyr Glu Lys Gly Leu Glu Phe Thr Pro Arg Phe Val
               20                  25                  30
   Asp Pro Thr Lys Phe Glu His His Glu Glu Trp Phe Lys Lys Ile Asn
           35                  40                  45
   Pro Arg Gly Gln Val Pro Ala Leu Asp His Asp Gly Asn Val Ile Thr
       50                  55                  60
   Glu Ser Thr Val Ile Cys Glu Tyr Leu Glu Asp Ala Phe Pro Asp Ala
   65                  70                  75                  80
   Pro Arg Leu Arg Pro Thr Asp Pro Val Gln Ile Ala Glu Met Arg Val
                   85                  90                  95
   Trp Thr Lys Trp Val Asp Glu Tyr Phe Cys Trp Cys Val Ser Thr Ile
               100                 105                 110
   Gly Trp Glu Arg Gly Ile Gly Pro Met Ala Arg Ala Leu Ser Asp Glu
           115                 120                 125
   Glu Phe Glu Glu Lys Val Lys Arg Ile Pro Ile Pro Glu Gln Gln Ala
       130                 135                 140
   Lys Trp Arg Ser Ala Arg Ala Gly Phe Pro Lys Glu Val Leu Asp Glu
   145                 150                 155                 160
   Glu Met Arg Lys Ile Arg Val Ser Ile Asp Arg Leu Glu Lys Arg Leu
                   165                 170                 175
   Ser Glu Ser Thr Trp Leu Ala Gly Glu Asp Tyr Thr Leu Ala Asp Ile
               180                 185                 190
   Cys Asn Phe Ala Ile Ala Asn Gly Met Glu Lys Gly Phe Asp Asp Ile
           195                 200                 205
   Val Asn Thr Ala Ala Thr Pro Asn Leu Val Ala Trp Ile Glu Arg Ile
           210                 215                 220
   Asn Ala Arg Pro Ala Cys Ile Glu Met Phe Ala Lys Ser Lys Ser Glu
   225                 230                 235                 240
   Phe Ala Ala Arg Lys Pro Phe Ala Lys Ser Glu Glu Gln Ala Gln Ala
                   245                 250                 255
<210> 4
   <211> 272
   <212> PRT
   <213> Artificial sequence
<220>
   <223> Enzyme
<400> 4
   Met Ala Lys Asp Asn Arg Ile Thr Leu Tyr Asp Leu Gln Leu Ala Ser
   1               5                   10                  15
   Gly Cys Thr Ile Ser Pro Phe Val Trp Arg Thr Lys Tyr Ala Leu Ala
               20                  25                  30
   His Lys Gly Phe Asp Met Asp Ile Val Pro Gly Gly Phe Thr Gly Ile
           35                  40                  45
   Ala Glu Arg Thr Gly Gly Arg Ser Glu Arg Ala Pro Val Ile Val Asp
       50                  55                  60
   Asp Gly Lys Trp Val Leu Asp Ser Trp Lys Ile Ala Glu Tyr Leu Asp
   65                  70                  75                  80
   Glu Thr Tyr Pro Asp Arg Pro Met Leu Phe Glu Gly Pro Ser Met Lys
                   85                  90                  95
   Val Leu Thr Lys Phe Leu Asp Ala Trp Leu Trp Lys Thr Ile Ile Ala
               100                 105                 110
   Pro Trp Phe Arg Cys Tyr Ile Leu Asp Tyr His Asp Leu Ser Leu Pro
           115                 120                 125
   Gln Asp His Ala Tyr Val Arg Glu Ser Arg Glu Thr Met Phe Leu Gly
       130                 135                 140
   Gly Gln Lys Leu Glu Asp Val Gln Ala Gly Arg Glu Asp Arg Leu Pro
   145                 150                 155                 160
   His Val Pro Pro Leu Leu Glu Pro Leu Arg Gln Leu Leu Arg Asp Thr
                   165                 170                 175
   Pro Trp Leu Gly Gly Ala Thr Pro Asn Tyr Ala Asp Tyr Thr Ala Leu
   180 185 190
   Ala Ile Phe Leu Trp Thr Gly Ser Val Cys Thr Thr Pro Pro Leu Thr
   195 200 205
   Glu Asp Asp Pro Leu Arg Asp Trp Leu Asp Arg Gly Phe Asp Leu Tyr
   210 215 220
   Gly Gly Leu Gly Arg His Pro Gly Met His Thr Leu Phe Gly Leu Lys
   225 230 235 240
   Leu Arg Glu Gly Asp Pro Glu Pro Phe Asp Arg Thr Gly Leu Gly Ile
   245 250 255
   Glu Pro Ala Pro Val Asn Gln Gly Ser Ala Glu Pro Ala Thr Ala Ser
   260 265 270
<210> 5
   <211> 270
   <212> PRT
   <213> Artificial sequence
<220>
   <223> Enzyme
<400> 5
   Met Tyr Gln Ile Pro Gly Cys Pro Phe Ser Glu Arg Val Glu Leu Leu
   1 5 10 15
   Leu Asp Leu Lys Gly Leu Gly Asp Val Leu Val Asp His Glu Ile Asp
   20 25 30
   Ile Ser Lys Pro Arg Pro Asp Trp Leu Leu Lys Lys Thr Arg Gly Thr
   35 40 45
   Thr Ser Leu Pro Ala Leu Glu Leu Glu Asn Gly Glu Thr Leu Lys Glu
   50 55 60
   Ser Met Val Ile Met Arg Tyr Ile Glu Asp Arg Phe Pro Glu Val Pro
   65 70 75 80
   Val Ala Arg Gln Asp Pro Tyr Glu His Ala Leu Glu Ala Met Leu Cys
   85 90 95
   Ala Thr Asp Gly Ala Tyr Thr Gly Ala Gly Tyr Arg Met Ile Leu Asn
   100 105 110
   Arg Asp Lys Ala Arg Arg Glu Glu Leu Lys Ala Glu Val Asp Ala Gln
           115                 120                 125
   Tyr Ala Arg Leu Asp Asp Phe Leu Arg His Tyr Ser Pro Asp Gly Val
       130                 135                 140
   Tyr Leu Phe Asp Arg Phe Gly Trp Ala Glu Val Ala Phe Ala Pro Met
   145                 150                 155                 160
   Phe Lys Arg Leu Trp Phe Leu Glu Tyr Tyr Glu Asp Tyr Glu Val Pro
                   165                 170                 175
   Gln Asn Leu Thr Arg Val Leu Leu Trp Arg Glu Ala Thr Leu Ala Glu
               180                 185                 190
   Pro Val Val Gln Ala Arg His Gly His Arg Glu Leu Met Thr Leu Tyr
           195                 200                 205
   Tyr Asp Tyr Thr Gln Gly Gly Gly Asn Gly Arg Leu Pro Glu Gly Arg
       210                 215                 220
   Ser Val Ser Ser Phe Thr Leu Asp Pro Pro Trp Arg Ala Arg Pro Met
   225                 230                 235                 240
   Pro Pro Arg Asp Lys Trp Gly Gln Gly Ala Ser Asp Ala Glu Leu Gly
                   245                 250                 255
   Leu Ile Pro Gly Ile Thr Val Arg Ser Asp Thr Val Pro Ala
               260                 265                 270
<210> 6
   <211> 209
   <212> PRT
   <213> Artificial sequence
<220>
   <223> Enzyme
<400> 6
   Met Leu Glu Glu Leu Asp Ala Asn Tyr Thr Leu Arg Pro Ile Ser Leu
   1               5                   10                  15
   Thr Asn Arg Glu Gln Lys Glu Asp Trp Tyr Leu Ala Arg Asn Pro Asn
               20                  25                  30
   Gly Arg Ile Pro Thr Leu Ile Asp His Glu Val Asp Ala Gly Asn Gly
           35                  40                  45
   Gly Phe Ala Val Phe Glu Ser Gly Ala Ile Leu Ile Tyr Leu Ala Glu
       50                  55                  60
   Lys Phe Gly Arg Phe Leu Pro Ala Asp Thr Met Gly Arg Ser Arg Ala
   65                  70                  75                  80
   Ile Gln Trp Val Met Trp Gln Met Ser Gly Leu Gly Pro Met Met Gly
                   85                  90                  95
   Gln Ala Thr Val Phe Asn Arg Tyr Phe Glu Pro Arg Leu Pro Glu Val
               100                 105                 110
   Ile Asp Arg Tyr Thr Arg Glu Ser Arg Arg Leu Phe Glu Val Met Asp
           115                 120                 125
   Thr His Leu Ala Asp Asn Glu Phe Leu Ala Gly Asp Tyr Ser Ile Ala
       130                 135                 140
   Asp Ile Ala Cys Phe Pro Trp Val Arg Gly His Asp Trp Ala Cys Ile
   145                 150                 155                 160
   Asp Met Glu Gly Leu Pro His Leu Gln Arg Trp Phe Glu Thr Ile Gly
                   165                 170                 175
   Glu Arg Pro Ala Val Gln Arg Gly Leu Leu Leu Pro Glu Pro Pro Lys
               180                 185                 190
   Ala Asp Glu Met Ala Glu Lys Thr Thr Arg Gln Gly Lys Asn Ile Leu
           195                 200                 205
   Ala
<210> 7
   <211> 912
   <212> DNA
   <213> Artificial sequence
<220>
   <223> Gene
<400> 7
   atgacacagg taaagggacg caccgcgttc atcactggcg gcggttcggg cgtggcgctc 60
   ggccaggcca aggttttcgc cagggctggc tgcaaggtcg ccattgccga catccgccag 120
   gatcacctcg acgaggccat ggcctggttc gaggccgaga acgccaaggg cgcgaactac 180
   gaggtgatgg cggtcaaact cgacatcacc gaccgcgagg cttacgccaa ggtcgccgac 240
   gaggtggaag cgaagctggg gcccgtcgaa ctgctgttca acaccgccgg ggtctcgcac 300
   ttcggcgcga tccaggatgc cacttacgac gactgggact ggcagatcga cgtcaacctg 360
   cgcggtgtga tcaacggcgt gcgcaccttc gtgccgcgca tgatcgagcg cggcaacggc 420
   ggccacgtgg tcaacaccgc ctcgatgtcg gccttcgtgg cgctcaaggg cacgggcatc 480
   tactgcacca ccaagatggc ggtgcgcggg ctgaccgaga ctctggccct cgatctggaa 540
   gagcatggca tcggcgtgtc gctgctgtgc ccgggcgcgg tcaacaccaa cattcacgaa 600
   gcgctgctga cccgccccaa gcatctggcg gacaccggct actaccaggc cgggccggag 660
   atgttcgcgc atctcaagaa cgtgatcgaa tgcggcatgg agcccgagac gctggcgaac 720
   cacgtcctga aggccgtgga ggagaaccag ctttacgttc tcgcctatcc ggaattccgc 780
   aagccgctgg aagacatcca cgcgcgcgtc atggccgccc tcgccaatcc cgaggacgat 840
   cccgactacg accggcgcgt ggcgcacggc gtaccgggcg gcgaggccaa ggaggaggaa 900
   aagacggcat ga 912
<210> 8
   <211> 936
   <212> DNA
   <213> Artificial sequence
<220>
   <223> Gene
<400> 8
   atgcaggatc taccggggaa gaccgccttt gtgaccggcg gcgccagcgg gattggcctg      60
   gggatagcca aggccctgct gggggcgggc atgaacgttg ctatcgccga cattcgccag     120
   gaccatctcg atgacgccgc agccgaactg gacggcggcg acaaggtgct cgcgctccag     180
   ctcgacgtca ccgatcgcgc tgcctttgcc gccgccgccg atgccaccga ggccaagttc     240
   ggcaagatcc acatcctgtg caacaacgca ggggttgcgg ttgtcggccc caccgacatg     300
   gcgactttcg ccgactggga ctgggtcatg ggcgtgaacg tgggcggcac gatcaacggc     360
   atcgtcacca tgctgccgcg catgttgaag cacggcgagg gcggccacat cgtcaacacc     420
   gcctcgatgt ccgcgctcgt gcccgcgccg ggcaccacga tctattcctc gggcaaagcc     480
   gccgtcacct cgatgatgga atgcatgcgc cccgaactgg aatcgcgcgg catcgtgtgc     540
   tcggccttct gcccaggcgc ggtgcagtcg aacattgccg aagccgggcg cacccgtccc     600
   gacgcgctgg ccgagaccgg ctacgccgag gccgacaagg ggcgccaggc ggggggcagt      660
   ttcttccacc tctaccagac caaggaagag gtcggtgagc gcgtgctgac gggcatcctc     720
   aacgatgaac tctacatcct cacccacgcc gagttcctca tcggcgtgca ggagcgcggc     780
   gaggcgacca ccgcggcggt gcaggtccag ttgcccgaga acgaggagta caagaacacc     840
   ttcggcgtgc tcttccgcaa ctcggcgatc acccaggaga tcgatcggca gaaggccctg     900
   cgcgccgcgc agatgtccga agccggcgtc gcgtaa                               936
<210> 9
   <211> 771
   <212> DNA
   <213> Artificial sequence
<220>
   <223> Gene
<400> 9
   atgttgacgc tgtacagctt tggccccgga gccaattcgc ttaagcccct gctggccctt 60
   tacgagaaag gcctcgaatt tacgccgcgc ttcgtcgatc cgaccaagtt cgagcatcac 120
   gaggaatggt tcaagaagat caacccgcgc ggccaggttc ccgcgctcga tcacgatggc 180
   aacgtcatca ccgaatcgac ggtgatttgc gaatacctcg aagacgcctt ccccgatgcg 240
   ccccggctgc gtcccaccga ccctgtgcag atcgccgaga tgcgggtctg gaccaagtgg 300
   gtggacgaat acttctgctg gtgcgtctcc accatcggct gggagcgcgg catcggtccg 360
   atggcccgtg ccctgtcgga cgaggagttc gaggaaaagg tcaaacgcat cccgatccct 420
   gagcagcagg ccaagtggcg cagcgcccgc gccggcttcc ccaaggaggt tctggacgag 480
   gaaatgcgca agatccgcgt ctcgatcgac cgtctcgaaa agcgcctttc cgaaagcacc 540
   tggctggcgg gcgaggacta tacgcttgcc gacatctgca acttcgccat cgccaacggc 600
   atggagaagg gctttgatga catcgtcaat acggccgcta cgcccaacct cgtcgcctgg 660
   atcgaacgca tcaacgcgcg tcccgcctgc atcgagatgt tcgccaagtc caagagcgag 720
   ttcgccgcgc gcaagccctt cgccaagagc gaagagcagg cacaggcctg a 771
<210> 10
   <211> 819
   <212> DNA
   <213> Artificial sequence
<220>
   <223> Gene
<400> 10
   atggccaagg acaaccgcat aacgctttac gatctgcagc ttgcctcggg ctgcacgatc 60
   agccccttcg tgtggcgcac caagtacgcg ctggcgcaca agggcttcga catggatatc 120
   gtgccgggcg gctttaccgg cattgccgag cgcacgggcg ggcgttccga acgtgcgcca 180
   gtgatcgtcg acgatggcaa gtgggtgctc gacagctgga agatcgccga atacctcgat 240
   gagacttatc ccgatcgccc gatgctgttc gagggccctt ccatgaaggt gctcaccaag 300
   ttcctcgacg cctggctatg gaagacgatc atcgcgccgt ggttccgctg ctacatcctc 360
   gactaccatg atctgtcgtt gccgcaggac catgcctacg tgcgcgaatc gcgcgagacg 420
   atgttcctgg gcgggcagaa gctggaggat gtgcaggcgg gccgcgagga tcggcttccg 480
   cacgtgccgc cgttgctgga gccgctgcgc cagctgctgc gcgatacgcc ctggctgggc 540
   ggtgcgacac ccaactacgc ggactacacc gcgctcgcca tcttcctgtg gaccggctct 600
   gtgtgcacca cgccgccgct caccgaggat gacccgttac gcgactggct cgaccgcggc 660
   ttcgaccttt acggcgggct ggggcgtcat ccgggcatgc acacgctgtt cggcctgaag 720
   ctgcgcgagg gcgatccaga gcctttcgac cgcaccggcc ttggcatcga gcccgcgccg 780
   gtcaaccagg gctcggccga gccggctacg gcgagctga 819
<210> 11
   <211> 813
   <212> DNA
   <213> Artificial sequence
<220>
   <223> Gene
<400> 11
   atgtaccaga tcccgggctg cccgttctcg gagcgggtgg agttgttgct cgatctcaag 60
   gggctgggcg atgtcctcgt cgaccacgag atcgacattt ccaaaccgcg cccggactgg 120
   ctcctgaaga agacgcgggg gacgacttcg cttcccgcgc tggagctgga gaacggggag 180
   accttgaagg agagcatggt catcatgcgc tacatcgagg accgcttccc cgaagttccg 240
   gtcgcgcgcc aggatcccta cgagcacgcc ctcgaggcga tgctgtgcgc gaccgatggc 300
   gcctacaccg gggcgggcta tcgcatgatc ctgaaccggg acaaggccag gcgcgaggaa 360
   ttgaaggccg aagtcgatgc gcagtacgcc cggctcgacg atttcctgcg gcactacagc 420
   ccggatgggg tctacctgtt cgaccgtttc ggctgggccg aagtggcctt tgcgccgatg 480
   ttcaagcggc tgtggttcct cgaatactac gaggactacg aggtgccgca gaacctgacg 540
   cgggtgcttc tgtggcgcga ggcgacactg gccgaacctg tcgtgcaggc gcgccacggc 600
   caccgcgagc tgatgacgct ctactacgac tacacccagg gcggtggaaa cggacgtctg 660
   cccgaagggc gcagcgtctc cagcttcacc ctcgatccgc cgtggcgcgc gcggcccatg 720
   ccgccgcgtg acaagtgggg gcagggcgcc agcgatgccg agctcgggct gatcccgggc 780
   attaccgtcc gttcggacac ggtgcccgcc tga 813
<210> 12
   <211> 630
   <212> DNA
   <213> Artificial sequence
<220>
   <223> Gene
<400> 12
   atgctcgagg agctggacgc gaactacacg ttgcgtccga tctcgctgac caaccgcgag 60
   cagaaggaag actggtatct cgcccgcaat cccaacgggc gtatccccac actgatcgac 120
   catgaggtcg atgccgggaa cggcggtttt gcggtgttcg aatcgggtgc gatcctgatc 180
   taccttgccg agaagttcgg ccgtttcctg ccagccgaca cgatgggccg cagccgcgcg 240
   atccagtggg tgatgtggca gatgtcgggc ctcggcccca tgatgggaca ggcgaccgtc 300
   ttcaaccgct acttcgagcc caggctgccc gaggtcatcg accgctacac gcgcgagagc 360
   cgccgcctct tcgaagtgat ggacacgcac ctcgccgaca acgaattcct cgcgggcgac 420
   tattcgatcg ccgacatcgc ctgcttcccg tgggtgcgcg ggcatgactg ggcctgcatc 480
   gacatggagg ggctgcccca cctgcaacgc tggttcgaga ccatcggtga gcgcccggcc 540
   gtccagcgcg gcctgctctt gcccgaaccg cccaaggcgg acgagatggc cgagaagacg 600
   acccgccagg gcaagaacat cctggcctga 630
<210> 13
   <211> 16
   <212> DNA
   <213> Artificial sequence
<220>
   <223> Primer
<400> 13
   tctcgagctc ggatcc 16
<210> 14
   <211> 18
   <212> DNA
   <213> Artificial sequence
<220>
   <223> Primer
<400> 14
   ctggtaccat ggaattcg 18
<210> 15
   <211> 35
   <212> DNA
   <213> Artificial sequence
<220>
   <223> Primer
<400> 15
   ggatccgagc tcgagatgat caagggtatc gaagg 35
<210> 16
   <211> 35
   <212> DNA
   <213> Artificial sequence
<220>
   <223> Primer
<400> 16
   cgaattccat ggtaccagtc agaactcctg tgcgg 35
<210> 17
   <211> 35
   <212> DNA
   <213> Artificial sequence
<220>
   <223> Primer
<400> 17
   ggatccgagc tcgagatgac gaactggctt atcac 35
<210> 18
   <211> 34
   <212> DNA
   <213> Artificial sequence
<220>
   <223> Primer
<400> 18
   cgaattccat ggtaccagtc agacctcggc gaag 34
<210> 19
   <211> 35
   <212> DNA
   <213> Artificial sequence
<220>
   <223> Primer
<400> 19
   ggatccgagc tcgagatgac acaggtaaag ggacg 35
<210> 20
   <211> 36
   <212> DNA
   <213> Artificial sequence
<220>
   <223> Primer
<400> 20
   cgaattccat ggtaccagtc atgccgtctt ttcctc 36
<210> 21
   <211> 34
   <212> DNA
   <213> Artificial sequence
<220>
   <223> Primer
<400> 21
   ggatccgagc tcgagatggg agagacgaca aaac 34
<210> 22
   <211> 33
   <212> DNA
   <213> Artificial sequence
<220>
   <223> Primer
<400> 22
   cgaattccat ggtaccagtc aggtgaggtc ggc 33
<210> 23
   <211> 32
   <212> DNA
   <213> Artificial sequence
<220>
   <223> Primer
<400> 23
   ggatccgagc tcgagatgca ggatctaccg gg 32
<210> 24
   <211> 32
   <212> DNA
   <213> Artificial sequence
<220>
   <223> Primer
<400> 24
   cgaattccat ggtaccgcaa gctgtgtcat gc 32
<210> 25
   <211> 28
   <212> DNA
   <213> Artificial sequence
<220>
   <223> Primer
<400> 25
   ggatccgagc tcgagatgac gggcgggg 28
<210> 26
   <211> 33
   <212> DNA
   <213> Artificial sequence
<220>
   <223> Primer
<400> 26
   cgaattccat ggtaccagtc agagcgcgtt ggc 33
<210> 27
   <211> 35
   <212> DNA
   <213> Artificial sequence
<220>
   <223> Primer
<400> 27
   ggatccgagc tcgagatgct ggaactgtgg acttc 35
<210> 28
   <211> 38
   <212> DNA
   <213> Artificial sequence
<220>
   <223> Primer
<400> 28
   cgaattccat ggtaccaggt aggtgtgctc atcgttca 38
<210> 29
   <211> 37
   <212> DNA
   <213> Artificial sequence
<220>
   <223> Primer
<400> 29
   ggatccgagc tcgagatgtt gacgctgtac agctttg 37
<210> 30
   <211> 34
   <212> DNA
   <213> Artificial sequence
<220>
   <223> Primer
<400> 30
   cgaattccat ggtaccagct cctcaggcct gtgc 34
<210> 31
   <211> 31
   <212> DNA
   <213> Artificial sequence
<220>
   <223> Primer
<400> 31
   ggatccgagc tcgagatggc caaggacaac c 31
<210> 32
   <211> 33
   <212> DNA
   <213> Artificial sequence
<220>
   <223> Primer
<400> 32
   cgaattccat ggtaccagtc agctcgccgt age 33
<210> 33
   <211> 31
   <212> DNA
   <213> Artificial sequence
<220>
   <223> Primer
<400> 33
   ggatccgagc tcgagatggc atgggacgat g 31
<210> 34
   <211> 36
   <212> DNA
   <213> Artificial sequence
<220>
   <223> Primer
<400> 34
   cgaattccat ggtaccagga tgacggtgtg cttcac 36